# EUROPEAN PATENT APPLICATION

(11) **EP 4 011 899 A1**
(43) Date of publication of application: **15.06.2022**
(21) Application number: 20850984.4
(22) Date of filing: 31.07.2020
(51) Int. Cl.: C07J 41/00, A61K 31/58, A61P 1/16

(54) **DEOXYCHOLIC ACID COMPOUNDS, PHARMACEUTICAL COMPOSITIONS AND USES THEREOF**

(30) Priority: 06.08.2019 CN 201910721398
(71) Applicant: Huang, Qiang, Suzhou, Jiangsu 215600 (CN)
(72) Inventor: DU, Jessica Xinyun, Redwood City, California 94065 (US)
(74) Representative: Bandpay & Greuter
(86) International application number: PCT/CN2020/106117
(87) International publication number: WO 2021/023100

(57) **Abstract**

Disclosed are a deoxycholic acid compound and a pharmaceutical composition thereof, and use thereof in the preparation of drugs for treating liver diseases. The structure of the deoxycholic acid compound is as shown by Formula **I,** or pharmaceutically acceptable salts thereof. The compounds have significantly liver-targeting characteristics, and the compounds reduce the drug concentration in the circulatory system while improving the efficacy, thereby reducing the toxic side effects.

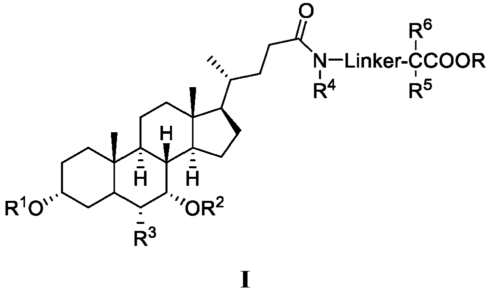

## Description

### TECHNICAL FIELD

The present disclosure relates to a deoxycholic acid compound, a pharmaceutical composition, and use thereof.

### BACKGROUND

Non-Alcohol Fatty Liver Diseases (NAFLDs) refer to a clinical pathological syndrome characterized mainly by excessive fat build-up in the liver cells caused by factors except for alcohol and other clear liver-damaging factors, which is an acquired metabolic stress liver injury closely associated with insulin resistance and genetic susceptibility. NAFLD includes simple fatty liver (SFL), non-alcoholic steatohepatitis (NASH), and associated cirrhoses. With the epidemic trend of globalization of obesity and obesity-associated metabolic syndrome, NAFLD has now become an important cause of chronic liver disease in developed countries such as Europe and the United States and in affluent regions of China. The prevalence of NAFLD in the general adult population is from 10% to 30%, of which 10% to 20% is NASH, with the latter having an incidence of cirrhosis of up to 25% within 10 years.

In addition to a direct cause of decompensated liver cirrhosis, hepatocellular carcinoma, and recurrence of transplanted livers, NAFLD can also affect the progression of other chronic liver diseases and be involved in the onset of type-2 diabetes and atherosclerosis. Metabolic syndrome-associated malignancies, atherosclerotic cardiovascular diseases and cirrhosis are important factors that affect the quality of life and life expectancy of patients with NAFLD. For this reason, NAFLD is a new challenge for the contemporary medical field. However, there is no effective method for treatment nowadays.

Deoxycholic acid compounds, such as chenodeoxycholic acid and 6-position substituted derivatives thereof, may activate farnesoid X receptor (FXR), and have anti-cholestatic and anti-fibrotic effects. FXR is a nuclear receptor, which is a bile acid sensor that may regulate the synthesis of bile acids and the flow of bile in the liver, and contribute to biliary homeostasis, lipid metabolism, glucose metabolism and inflammatory/immune response. Research data suggests that 6-α-ethylchenodeoxycholic acid (obeticholic acid, OCA) is useful for treatment of primary biliary cirrhosis (PBC), portal hypertension, non-alcoholic steatohepatitis (NASH), bile acid diarrhea, alcoholic hepatitis, primary sclerosing cholangitis Bile acid diarrhea, alcoholic hepatitis, primary sclerosing cholangitis (PSC), and other bile secretion-associated diseases (Drug Discovery Today. Volume 17, Numbers 17/18, 2012).

However, when the obeticholic acid is used in the clinical treatment of liver diseases such as fatty liver, the incidence of pruritus reaches 63%, and the incidence of rash is also up to 32%, so it must be used sometimes in combination with other drugs to control the toxic effects of the obeticholic acid on the skin. This causes the obeticholic acid to be clinically limited.

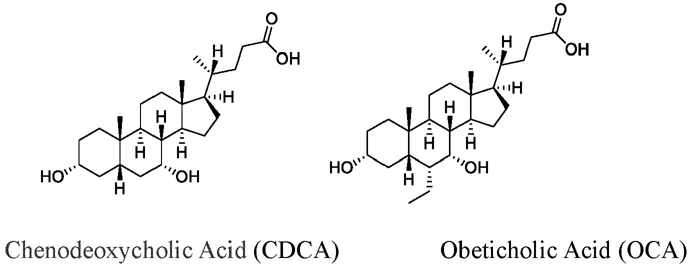

### Deoxycholic Acid Compound

Obeticholic acid (OCA) is mainly metabolized *in vivo* with glycine (Gly) and taurine (Tau) to produce metabolic conjugates OCA-Gly and OCA-Tau, respectively. Studies have shown that the above-mentioned metabolic conjugates of the obeticholic acid exhibit the same physiological effects as those of the obeticholic acid, for example, their effects on activation of the farnesoid X receptor (FXR) activity are almost the same as the effect of the obeticholic acid (reference literature: Obeticholic acid, a selective farnesoid X receptor agonist, regulates bile acid homeostasis in sandwich-cultured human hepatocytes. Pharmacology Research & Perspectives. 2017, vol. 5, iss.4).

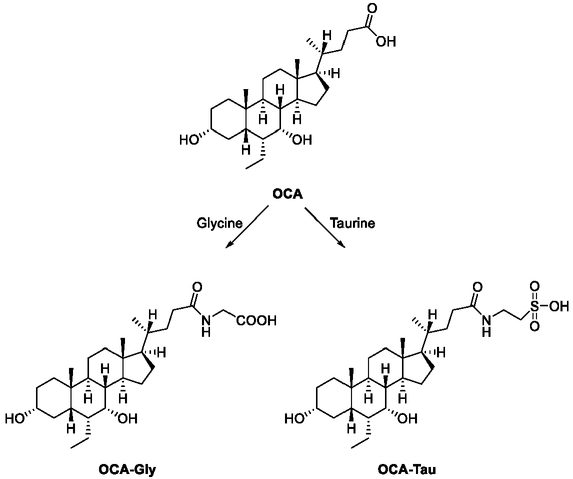

*In vivo* metabolism of obeticholic acid and metabolite thereof

### SUMMARY

Inventors have unexpectedly discovered that a class of derivatives of the obeticholic acid-glycine (OCA-Gly) conjugate has a significant liver-targeting characteristic, and the active drug concentration in liver is about 20 times that in plasma. Therefore, when such compounds are used to treat liver diseases such as fatty liver, the drug efficacy is improved due to a significant increase of the active drug concentration in the liver, and the side effects such as rashes are reduced due to a significant decrease of the effective drug concentration in the plasma.

In view of the above, the present disclosure provides a compound of Formula **I:** 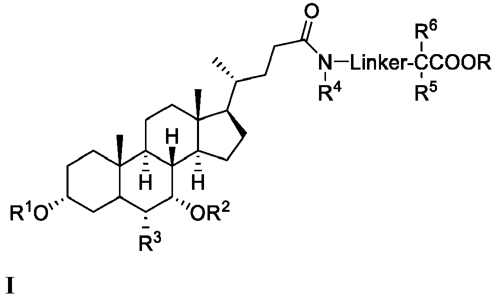 or a pharmaceutically acceptable salt thereof, wherein:
R¹ and R² each are independently selected from the group consisting of: H, -COR', -CONHR', -CONR'R", or -COOR';
R³ is selected from the group consisting of: H, methyl, or ethyl;
R⁴ is selected from the group consisting of: H, alkyl or substituted alkyl, alkenyl or substituted alkenyl, alkynyl or substituted alkynyl, cycloalkyl or substituted cycloalkyl, aryl or substituted aryl, heteroaryl or substituted heteroaryl, and heterocyclyl or substituted heterocyclyl;
Linker is absent, or is selected from the group consisting of: alkylene or substituted alkylene, alkenylene or substituted alkenylene, alkynylene or substituted alkynylene, cycloalkylene or substituted cycloalkylene, arylene or substituted arylene, heteroarylene or substituted heteroarylene, and heterocyclylene or substituted heterocyclylene;
R⁵ and R⁶ each are independently selected from the group consisting of: H, and alkyl or substituted alkyl; or R⁵ and R⁶ are linked to each other, and form a 3-7 membered ring with 0, 1, or 2 heteroatoms selected from the group consisting of O, S, or N, together with C atoms linked thereto;
R is selected from the group consisting of: alkyl or substituted alkyl, alkenyl or substituted alkenyl, alkynyl or substituted alkynyl, cycloalkyl or substituted cycloalkyl, aryl or substituted aryl, alkylene-aryl, alkylene-substituted aryl, heteroaryl or substituted heteroaryl, and heterocyclyl or substituted heterocyclyl; or
R is selected from the group consisting of: inorganic cations selected from the group consisting of sodium ions, potassium ions, magnesium ions, or calcium ions; or organic cations selected from the group consisting of ammonium, tetramethylammonium, tetraethylammonium, tetrapropylammonium, or tetrabutyl ammonium;
R' and R" each are independently selected from the group consisting of: H, alkyl or substituted alkyl, alkenyl or substituted alkenyl, alkynyl or substituted alkynyl, cycloalkyl or substituted cycloalkyl, aryl or substituted aryl, heteroaryl or substituted heteroaryl, and heterocyclyl or substituted heterocyclyl; and
substituents of the substituted alkyl, the substituted alkylene, the substituted alkenyl, the substituted alkenylene, the substituted alkynyl, the substituted alkynylene, the substituted cycloalkyl, the substituted cycloalkylene, the substituted aryl, the substituted arylene, the alkylene-substituted aryl, the substituted heteroaryl, the substituted heteroarylene, the substituted heterocyclyl, or the substituted heterocyclylene each are independently selected from the group consisting of: halogen, cyano, amino, nitro, hydroxy, alkyl, alkoxyl, and modified alkyl.

Optionally, the compound of Formula **I** is not a compound: 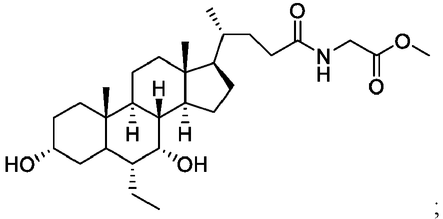 optionally, R in the compound of Formula **I** is not methyl.

Optionally, the "halogen" is selected from the group consisting of: F, Cl, Br, or I;
optionally, "alkyl" in the "alkyl" and "alkoxyl" is C₁-C₂₀ linear or branched alkyl, optionally selected from the group consisting of: methyl, ethyl, n-propyl, isopropyl, n-butyl, isobutyl, tert-butyl, sec-butyl, and n-pentyl;
optionally, "alkylene" in the "alkylene", "alkylene-aryl", and "alkylene-substituted aryl" is C₁-C₁₀ linear or branched alkylene, optionally selected from the group consisting of: methylene, ethylene, n-propylidene, isopropylidene, n-butylidene, isobutylidene, tert-butylidene, sec-butylidene, and n-pentylene;
optionally, the "modified alkyl" is a group obtained by substituting one or more groups selected from the group consisting of -O-, -CO-, -NH₂, -OH, halogen, -CN, and -NO₂ for any carbon atom in the alkyl;
optionally, the "alkenyl" is C₂-C₆ alkenyl;
optionally, the "alkenylene" is C₂-C₆ alkenylene;
optionally, the "alkynyl" is C₂-C₆ alkynyl;
optionally, the "alkynylene" is C₂-C₆ alkynylene;
optionally, the "cycloalkyl" is C₃-C₁₀ monocyclic or bicyclic cycloalkyl;
optionally, the "cycloalkylene" is C₃-C₁₀ monocyclic or bicyclic cycloalkylene;
optionally, "aryl" in the "aryl", the "alkylene-aryl", and the "alkylene-substituted aryl" is 6-10 membered aryl; and is optionally phenyl or naphthyl;
optionally, the "arylene" is 6-10 membered arylene; and may be optionally phenylene or naphthylene;
optionally, the "heteroaryl" is a 5-10 membered heteroaromatic ring containing 1, 2, or 3 heteroatoms selected from the group consisting of N, O, and S;
optionally, the "heteroarylene" is a 5-10 membered heteroarylene ring containing 1, 2, or 3 heteroatoms selected from the group consisting of N, O, and S;
optionally, the "heterocyclyl" is a 3-10 membered nonaromatic heterocyclic ring containing 1, 2, or 3 heteroatoms selected from the group consisting of N, O, and S; and
optionally, the "heterocyclylene" is a 3-10 membered nonaromatic heterocyclylene ring containing 1, 2, or 3 heteroatoms selected from the group consisting of N, O, and S.

Optionally, R' and R" each are independently selected from the group consisting of: H, methyl, ethyl, n-propyl, isopropyl, n-butyl, isobutyl, benzyl, sec-butyl, n-pentyl, 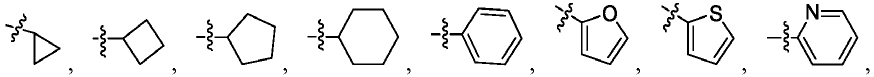 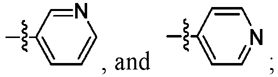
optionally, R¹ and R² each are independently selected from the group consisting of: H, acetyl, propionyl, isopropionyl, butyryl, and isobutyryl;
optionally, R³ is selected from the group consisting of: H, methyl, or ethyl;
optionally, R⁴ is selected from the group consisting of: methyl, ethyl, n-propyl, isopropyl, n-butyl, isobutyl, tert-butyl, sec-butyl, and n-pentyl;
optionally, Linker is absent, or is selected from the group consisting of: -CH₂-, - CH₂CH₂-, -CH₂CH₂CH₂-, cyclopropylidene, 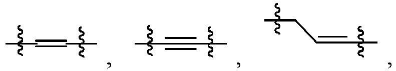 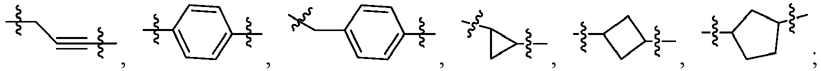
optionally, R⁵ and R⁶ each are independently selected from the group consisting of: methyl, ethyl, n-propyl, isopropyl, n-butyl, isobutyl, tert-butyl, sec-butyl, n-pentyl, or R⁵ and R⁶ are linked to each other, and form, together with the C atoms linked thereto, any group selected from the following formulas: 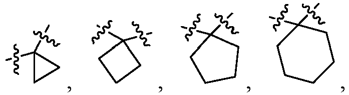 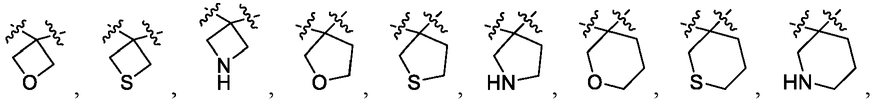 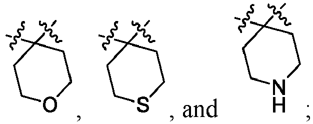 and
optionally, R is selected from the group consisting of: ethyl, propyl, butyl, isopropyl, isobutyl, tert-butyl, sec-butyl, n-pentyl, 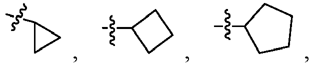 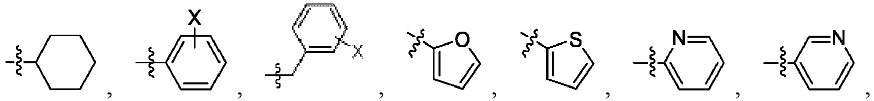 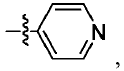 heterocyclyl or substituted heterocyclyl, sodium ions, potassium ions, magnesium ions, calcium ions, ammonium, tetramethylammonium, tetraethylammonium, tetrapropylammonium, or tetrabutylammonium; and Xs each are independently selected from the group consisting of: 0, 1, 2, or 3 groups selected from the group consisting of: F, Cl, Br, I, -NH₂, -OH, -methoxyl, ethoxyl, propoxyl, isopropoxyl, -CN, methyl, ethyl, n-propyl, isopropyl, n-butyl, or isobutyl, or Xs each are independently a group obtained by substituting one or more groups selected from the group consisting of -O-, -CO-, and -NH₂ for any carbon atom on C₅-C₁₀ linear or branched alkyl.

Optionally, the compound of Formula **I** is selected from a compound of the following Formula **II**: 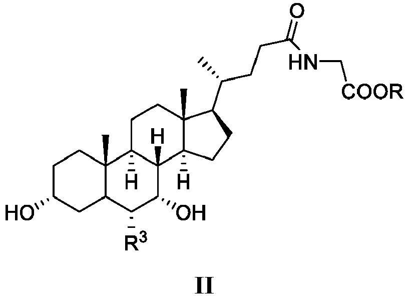 wherein:
R³ and R are as defined above.

Optionally, in Formula **II**, R³ is selected from the group consisting of: H, methyl, or ethyl; R is selected from the group consisting of: ethyl, n-propyl, isopropyl, n-butyl, isobutyl, 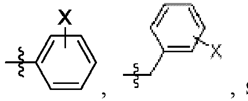 sodium ions, potassium ions, calcium ions, and Xs each are independently selected from the group consisting of: 0, 1, 2, or 3 groups selected from the group consisting of: F, Cl, Br, I, -NH₂, -OH, -methoxyl, ethoxyl, propoxyl, isopropoxyl, -CN, methyl, ethyl, n-propyl, isopropyl, n-butyl, or isobutyl, or Xs each are independently a group obtained by substituting one or more groups selected from the group consisting of -O-, -CO-, and -NH₂ for any carbon atom on C₅-C₁₀ linear or branched alkyl.

Optionally, the compound of Formula **I** or the pharmaceutically acceptable salt thereof is selected from the following compounds: 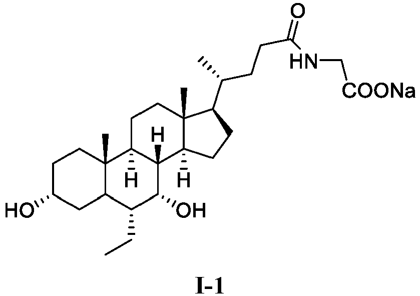 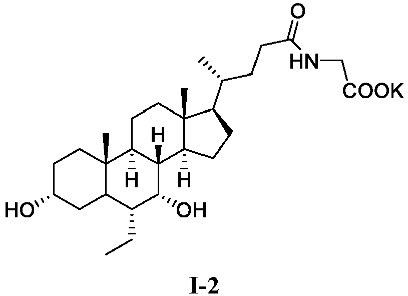 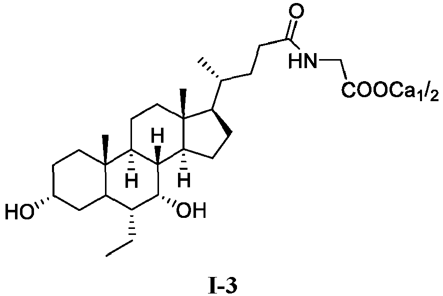 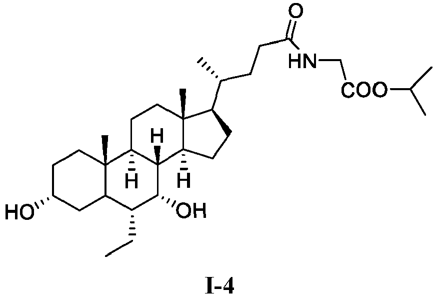 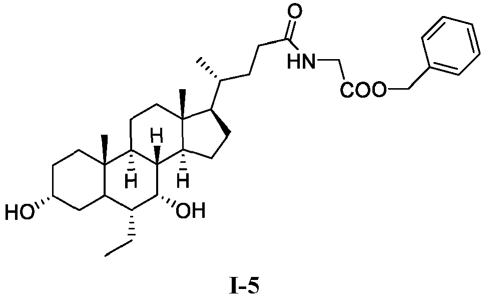 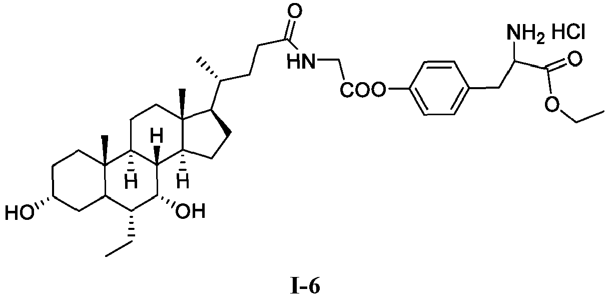 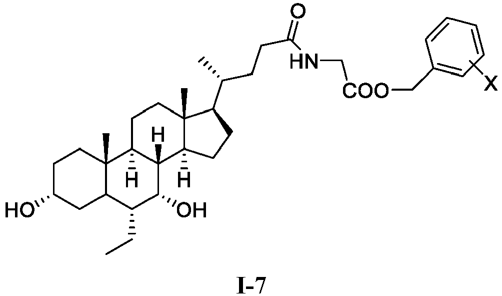
in Formula **I-7,** X is 1, 2, or 3 substituents, each independently selected from the group consisting of F, Cl, Br, I, -CN, -NH₂, -NO₂, or -OH, methyl, ethyl, propyl, isopropyl, methoxyl, ethoxyl, propoxyl, and isopropoxyl; and 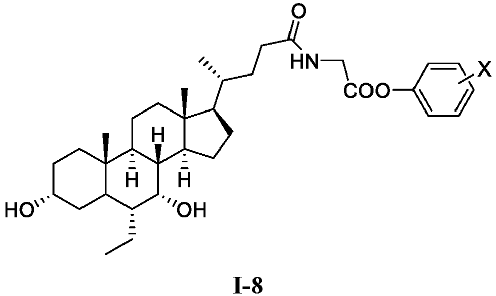
in Formula **I-8,** X is 1, 2, or 3 substituents, each independently selected from the group consisting of F, Cl, Br, I, -CN, -NH₂, -NO₂, or -OH, methyl, ethyl, propyl, isopropyl, methoxyl, ethoxyl, propoxyl, and isopropoxyl.

Optionally, the compound of Formula **I** is selected from the compounds listed in the table below:

| R³ | R¹ | R² | R⁴ | Linker | R⁵ | R⁶ | R |
|---|---|---|---|---|---|---|---|
| Et | -H | -H | -H | Absence | -H | -H | -Et, -iPr, -Bn, -Ph |
| Et | -H | -H | -H | Absence | -H | -CH₃ | -Et, -iPr, -Bn, -Ph |
| Et | -H | -H | -H | Absence | -H | -Et | -Et, -iPr, -Bn, -Ph |
| Et | -H | -H | -H | Absence | -H | -Pr | -Et, -iPr, -Bn, -Ph |
| Et | -H | -H | -H | Absence | -H | -iPr | -Et, -iPr, -Bn, -Ph |
| Et | -H | -H | -H | Absence | -H | -Bu | -Et, -iPr, -Bn, -Ph |
| Et | -H | -H | -H | Absence | -H | -CH₂iPr | -Et, -iPr, -Bn, -Ph |
| Et | -H | -H | -H | Absence | -H | -CH(Me)Et | -Et, -iPr, -Bn, -Ph |
| Et | -H | -H | -H | Absence | -H | -nPentyl | -Et, -iPr, -Bn, -Ph |
| Et | -H | -H | -H | Absence | -H | -CH(Me)Pr | -Et, -iPr, -Bn, -Ph |
| Et | -H | -H | -H | Absence | -H | -CH(Me)iPr | -Et, -iPr, -Bn, -Ph |
| Et | -H | -H | -H | Absence | -H | -CH₂tBu | -Et, -iPr, -Bn, -Ph |
| Et | -Ac | -H | -H | Absence | -H | -H | -Et, -iPr, -Bn, -Ph |
| Et | -Ac | -H | -H | Absence | -H | -CH₃ | -Et, -iPr, -Bn, -Ph |
| Et | -Ac | -H | -H | Absence | -H | -Et | -Et, -iPr, -Bn, -Ph |
| Et | -Ac | -H | -H | Absence | -H | -Pr | -Et, -iPr, -Bn, -Ph |
| Et | -Ac | -H | -H | Absence | -H | -iPr | -Et, -iPr, -Bn, -Ph |
| Et | -Ac | -H | -H | Absence | -H | -Bu | -Et, -iPr, -Bn, -Ph |
| Et | -Ac | -H | -H | Absence | -H | -CH₂iPr | -Et, -iPr, -Bn, -Ph |
| Et | -Ac | -H | -H | Absence | -H | -CH(Me)Et | -Et, -iPr, -Bn, -Ph |
| Et | -Ac | -H | -H | Absence | -H | -nPentyl | -Et, -iPr, -Bn, -Ph |
| Et | -Ac | -H | -H | Absence | -H | -CH(Me)Pr | -Et, -iPr, -Bn, -Ph |
| Et | -Ac | -H | -H | Absence | -H | -CH(Me)iPr | -Et, -iPr, -Bn, -Ph |
| Et | -Ac | -H | -H | Absence | -H | -CH₂tBu | -Et, -iPr, -Bn, -Ph |
| Et | -H | -Ac | -H | Absence | -H | -H | -Et, -iPr, -Bn, -Ph |
| Et | -H | -Ac | -H | Absence | -H | -CH₃ | -Et, -iPr, -Bn, -Ph |
| Et | -H | -Ac | -H | Absence | -H | -Et | -Et, -iPr, -Bn, -Ph |
| Et | -H | -Ac | -H | Absence | -H | -Pr | -Et, -iPr, -Bn, -Ph |
| Et | -H | -Ac | -H | Absence | -H | -iPr | -Et, -iPr, -Bn, -Ph |
| Et | -H | -Ac | -H | Absence | -H | -Bu | -Et, -iPr, -Bn, -Ph |
| Et | -H | -Ac | -H | Absence | -H | -CH₂iPr | -Et, -iPr, -Bn, -Ph |
| Et | -H | -Ac | -H | Absence | -H | -CH(Me)Et | -Et, -iPr, -Bn, -Ph |
| Et | -H | -Ac | -H | Absence | -H | -nPentyl | -Et, -iPr, -Bn, -Ph |
| Et | -H | -Ac | -H | Absence | -H | -CH(Me)Pr | -Et, -iPr, -Bn, -Ph |
| Et | -H | -Ac | -H | Absence | -H | -CH(Me)iPr | -Et, -iPr, -Bn, -Ph |
| Et | -H | -Ac | -H | Absence | -H | -CH₂tBu | -Et, -iPr, -Bn, -Ph |
| Et | -Ac | -Ac | -H | Absence | -H | -H | -Et, -iPr, -Bn, -Ph |
| Et | -Ac | -Ac | -H | Absence | -H | -CH₃ | -Et, -iPr, -Bn, -Ph |
| Et | -Ac | -Ac | -H | Absence | -H | -Et | -Et, -iPr, -Bn, -Ph |
| Et | -Ac | -Ac | -H | Absence | -H | -Pr | -Et, -iPr, -Bn, -Ph |
| Et | -Ac | -Ac | -H | Absence | -H | -iPr | -Et, -iPr, -Bn, -Ph |
| Et | -Ac | -Ac | -H | Absence | -H | -Bu | -Et, -iPr, -Bn, -Ph |
| Et | -Ac | -Ac | -H | Absence | -H | -CH₂iPr | -Et, -iPr, -Bn, -Ph |
| Et | -Ac | -Ac | -H | Absence | -H | -CH(Me)Et | -Et, -iPr, -Bn, -Ph |
| Et | -Ac | -Ac | -H | Absence | -H | -nPentyl | -Et, -iPr, -Bn, -Ph |
| Et | -Ac | -Ac | -H | Absence | -H | -CH(Me)Pr | -Et, -iPr, -Bn, -Ph |
| Et | -Ac | -Ac | -H | Absence | -H | -CH(Me)iPr | -Et, -iPr, -Bn, -Ph |
| Et | -Ac | -Ac | -H | Absence | -H | -CH₂tBu | -Et, -iPr, -Bn, -Ph |
| Et | -COEt | -H | -H | Absence | -H | -H | -Et, -iPr, -Bn, -Ph |
| Et | -COEt | -H | -H | Absence | -H | -CH₃ | -Et, -iPr, -Bn, -Ph |
| Et | -COEt | -H | -H | Absence | -H | -Et | -Et, -iPr, -Bn, -Ph |
| Et | -COEt | -H | -H | Absence | -H | -Pr | -Et, -iPr, -Bn, -Ph |
| Et | -COEt | -H | -H | Absence | -H | -iPr | -Et, -iPr, -Bn, -Ph |
| Et | -COEt | -H | -H | Absence | -H | -Bu | -Et, -iPr, -Bn, -Ph |
| Et | -COEt | -H | -H | Absence | -H | -CH₂iPr | -Et, -iPr, -Bn, -Ph |
| Et | -COEt | -H | -H | Absence | -H | -CH(Me)Et | -Et, -iPr, -Bn, -Ph |
| Et | -COEt | -H | -H | Absence | -H | -nPentyl | -Et, -iPr, -Bn, -Ph |
| Et | -COEt | -H | -H | Absence | -H | -CH(Me)Pr | -Et, -iPr, -Bn, -Ph |
| Et | -COEt | -H | -H | Absence | -H | -CH(Me)iPr | -Et, -iPr, -Bn, -Ph |
| Et | -COEt | -H | -H | Absence | -H | -CH₂tBu | -Et, -iPr, -Bn, -Ph |
| Et | -H | -COEt | -H | Absence | -H | -H | -Et, -iPr, -Bn, -Ph |
| Et | -H | -COEt | -H | Absence | -H | -CH₃ | -Et, -iPr, -Bn, -Ph |
| Et | -H | -COEt | -H | Absence | -H | -Et | -Et, -iPr, -Bn, -Ph |
| Et | -H | -COEt | -H | Absence | -H | -Pr | -Et, -iPr, -Bn, -Ph |
| Et | -H | -COEt | -H | Absence | -H | -iPr | -Et, -iPr, -Bn, -Ph |
| Et | -H | -COEt | -H | Absence | -H | -Bu | -Et, -iPr, -Bn, -Ph |
| Et | -H | -COEt | -H | Absence | -H | -CH₂iPr | -Et, -iPr, -Bn, -Ph |
| Et | -H | -COEt | -H | Absence | -H | -CH(Me)Et | -Et, -iPr, -Bn, -Ph |
| Et | -H | -COEt | -H | Absence | -H | -nPentyl | -Et, -iPr, -Bn, -Ph |
| Et | -H | -COEt | -H | Absence | -H | -CH(Me)Pr | -Et, -iPr, -Bn, -Ph |
| Et | -H | -COEt | -H | Absence | -H | -CH(Me)iPr | -Et, -iPr, -Bn, -Ph |
| Et | -H | -COEt | -H | Absence | -H | -CH₂tBu | -Et, -iPr, -Bn, -Ph |
| Et | -COEt | -COEt | -H | Absence | -H | -H | -Et, -iPr, -Bn, -Ph |
| Et | -COEt | -COEt | -H | Absence | -H | -CH₃ | -Et, -iPr, -Bn, -Ph |
| Et | -COEt | -COEt | -H | Absence | -H | -Et | -Et, -iPr, -Bn, -Ph |
| Et | -COEt | -COEt | -H | Absence | -H | -Pr | -Et, -iPr, -Bn, -Ph |
| Et | -COEt | -COEt | -H | Absence | -H | -iPr | -Et, -iPr, -Bn, -Ph |
| Et | -COEt | -COEt | -H | Absence | -H | -Bu | -Et, -iPr, -Bn, -Ph |
| Et | -COEt | -COEt | -H | Absence | -H | -CH₂iPr | -Et, -iPr, -Bn, -Ph |
| Et | -COEt | -COEt | -H | Absence | -H | -CH(Me)Et | -Et, -iPr, -Bn, -Ph |
| Et | -COEt | -COEt | -H | Absence | -H | -nPentyl | -Et, -iPr, -Bn, -Ph |
| Et | -COEt | -COEt | -H | Absence | -H | -CH(Me)Pr | -Et, -iPr, -Bn, -Ph |
| Et | -COEt | -COEt | -H | Absence | -H | -CH(Me)iPr | -Et, -iPr, -Bn, -Ph |
| Et | -COEt | -COEt | -H | Absence | -H | -CH₂tBu | -Et, -iPr, -Bn, -Ph |
| Et | -COPr | -H | -H | Absence | -H | -H | -Et, -iPr, -Bn, -Ph |
| Et | -COPr | -H | -H | Absence | -H | -CH₃ | -Et, -iPr, -Bn, -Ph |
| Et | -COPr | -H | -H | Absence | -H | -Et | -Et, -iPr, -Bn, -Ph |
| Et | -COPr | -H | -H | Absence | -H | -Pr | -Et, -iPr, -Bn, -Ph |
| Et | -COPr | -H | -H | Absence | -H | -iPr | -Et, -iPr, -Bn, -Ph |
| Et | -COPr | -H | -H | Absence | -H | -Bu | -Et, -iPr, -Bn, -Ph |
| Et | -COPr | -H | -H | Absence | -H | -CH₂iPr | -Et, -iPr, -Bn, -Ph |
| Et | -COPr | -H | -H | Absence | -H | -CH(Me)Et | -Et, -iPr, -Bn, -Ph |
| Et | -COPr | -H | -H | Absence | -H | -nPentyl | -Et, -iPr, -Bn, -Ph |
| Et | -COPr | -H | -H | Absence | -H | -CH(Me)Pr | -Et, -iPr, -Bn, -Ph |
| Et | -COPr | -H | -H | Absence | -H | -CH(Me)iPr | -Et, -iPr, -Bn, -Ph |
| Et | -COPr | -H | -H | Absence | -H | -CH₂tBu | -Et, -iPr, -Bn, -Ph |
| Et | -H | -COPr | -H | Absence | -H | -H | -Et, -iPr, -Bn, -Ph |
| Et | -H | -COPr | -H | Absence | -H | -CH₃ | -Et, -iPr, -Bn, -Ph |
| Et | -H | -COPr | -H | Absence | -H | -Et | -Et, -iPr, -Bn, -Ph |
| Et | -H | -COPr | -H | Absence | -H | -Pr | -Et, -iPr, -Bn, -Ph |
| Et | -H | -COPr | -H | Absence | -H | -iPr | -Et, -iPr, -Bn, -Ph |
| Et | -H | -COPr | -H | Absence | -H | -Bu | -Et, -iPr, -Bn, -Ph |
| Et | -H | -COPr | -H | Absence | -H | -CH₂iPr | -Et, -iPr, -Bn, -Ph |
| Et | -H | -COPr | -H | Absence | -H | -CH(Me)Et | -Et, -iPr, -Bn, -Ph |
| Et | -H | -COPr | -H | Absence | -H | -nPentyl | -Et, -iPr, -Bn, -Ph |
| Et | -H | -COPr | -H | Absence | -H | -CH(Me)Pr | -Et, -iPr, -Bn, -Ph |
| Et | -H | -COPr | -H | Absence | -H | -CH(Me)iPr | -Et, -iPr, -Bn, -Ph |
| Et | -H | -COPr | -H | Absence | -H | -CH₂tBu | -Et, -iPr, -Bn, -Ph |
| Et | -COPr | -COPr | -H | Absence | -H | -H | -Et, -iPr, -Bn, -Ph |
| Et | -COPr | -COPr | -H | Absence | -H | -CH₃ | -Et, -iPr, -Bn, -Ph |
| Et | -COPr | -COPr | -H | Absence | -H | -Et | -Et, -iPr, -Bn, -Ph |
| Et | -COPr | -COPr | -H | Absence | -H | -Pr | -Et, -iPr, -Bn, -Ph |
| Et | -COPr | -COPr | -H | Absence | -H | -iPr | -Et, -iPr, -Bn, -Ph |
| Et | -COPr | -COPr | -H | Absence | -H | -Bu | -Et, -iPr, -Bn, -Ph |
| Et | -COPr | -COPr | -H | Absence | -H | -CH₂iPr | -Et, -iPr, -Bn, -Ph |
| Et | -COPr | -COPr | -H | Absence | -H | -CH(Me)Et | -Et, -iPr, -Bn, -Ph |
| Et | -COPr | -COPr | -H | Absence | -H | -nPentyl | -Et, -iPr, -Bn, -Ph |
| Et | -COPr | -COPr | -H | Absence | -H | -CH(Me)Pr | -Et, -iPr, -Bn, -Ph |
| Et | -COPr | -COPr | -H | Absence | -H | -CH(Me)iPr | -Et, -iPr, -Bn, -Ph |
| Et | -COPr | -COPr | -H | Absence | -H | -CH₂tBu | -Et, -iPr, -Bn, -Ph |
| Et | -COiPr | -H | -H | Absence | -H | -H | -Et, -iPr, -Bn, -Ph |
| Et | -COiPr | -H | -H | Absence | -H | -CH₃ | -Et, -iPr, -Bn, -Ph |
| Et | -COiPr | -H | -H | Absence | -H | -Et | -Et, -iPr, -Bn, -Ph |
| Et | -COiPr | -H | -H | Absence | -H | -Pr | -Et, -iPr, -Bn, -Ph |
| Et | -COiPr | -H | -H | Absence | -H | -iPr | -Et, -iPr, -Bn, -Ph |
| Et | -COiPr | -H | -H | Absence | -H | -Bu | -Et, -iPr, -Bn, -Ph |
| Et | -COiPr | -H | -H | Absence | -H | -CH₂iPr | -Et, -iPr, -Bn, -Ph |
| Et | -COiPr | -H | -H | Absence | -H | -CH(Me)Et | -Et, -iPr, -Bn, -Ph |
| Et | -COiPr | -H | -H | Absence | -H | -nPentyl | -Et, -iPr, -Bn, -Ph |
| Et | -COiPr | -H | -H | Absence | -H | -CH(Me)Pr | -Et, -iPr, -Bn, -Ph |
| Et | -COiPr | -H | -H | Absence | -H | -CH(Me)iPr | -Et, -iPr, -Bn, -Ph |
| Et | -COiPr | -H | -H | Absence | -H | -CH₂tBu | -Et, -iPr, -Bn, -Ph |
| Et | -H | -COiPr | -H | Absence | -H | -H | -Et, -iPr, -Bn, -Ph |
| Et | -H | -COiPr | -H | Absence | -H | -CH₃ | -Et, -iPr, -Bn, -Ph |
| Et | -H | -COiPr | -H | Absence | -H | -Et | -Et, -iPr, -Bn, -Ph |
| Et | -H | -COiPr | -H | Absence | -H | -Pr | -Et, -iPr, -Bn, -Ph |
| Et | -H | -COiPr | -H | Absence | -H | -iPr | -Et, -iPr, -Bn, -Ph |
| Et | -H | -COiPr | -H | Absence | -H | -Bu | -Et, -iPr, -Bn, -Ph |
| Et | -H | -COiPr | -H | Absence | -H | -CH₂iPr | -Et, -iPr, -Bn, -Ph |
| Et | -H | -COiPr | -H | Absence | -H | -CH(Me)Et | -Et, -iPr, -Bn, -Ph |
| Et | -H | -COiPr | -H | Absence | -H | -nPentyl | -Et, -iPr, -Bn, -Ph |
| Et | -H | -COiPr | -H | Absence | -H | -CH(Me)Pr | -Et, -iPr, -Bn, -Ph |
| Et | -H | -COiPr | -H | Absence | -H | -CH(Me)iPr | -Et, -iPr, -Bn, -Ph |
| Et | -H | -COiPr | -H | Absence | -H | -CH₂tBu | -Et, -iPr, -Bn, -Ph |
| Et | -COiPr | -COiPr | -H | Absence | -H | -H | -Et, -iPr, -Bn, -Ph |
| Et | -COiPr | -COiPr | -H | Absence | -H | -CH₃ | -Et, -iPr, -Bn, -Ph |
| Et | -COiPr | -COiPr | -H | Absence | -H | -Et | -Et, -iPr, -Bn, -Ph |
| Et | -COiPr | -COiPr | -H | Absence | -H | -Pr | -Et, -iPr, -Bn, -Ph |
| Et | -COiPr | -COiPr | -H | Absence | -H | -iPr | -Et, -iPr, -Bn, -Ph |
| Et | -COiPr | -COiPr | -H | Absence | -H | -Bu | -Et, -iPr, -Bn, -Ph |
| Et | -COiPr | -COiPr | -H | Absence | -H | -CH₂iPr | -Et, -iPr, -Bn, -Ph |
| Et | -COiPr | -COiPr | -H | Absence | -H | -CH(Me)Et | -Et, -iPr, -Bn, -Ph |
| Et | -COiPr | -COiPr | -H | Absence | -H | -nPentyl | -Et, -iPr, -Bn, -Ph |
| Et | -COiPr | -COiPr | -H | Absence | -H | -CH(Me)Pr | -Et, -iPr, -Bn, -Ph |
| Et | -COiPr | -COiPr | -H | Absence | -H | -CH(Me)iPr | -Et, -iPr, -Bn, -Ph |
| Et | -COiPr | -COiPr | -H | Absence | -H | -CH₂tBu | -Et, -iPr, -Bn, -Ph |

Optionally, the pharmaceutically acceptable salt includes salts of the compound of Formula **I.** These cationic salts include salts of alkali metals, salts of alkaline earth metals, and ammonium salts. Optionally, the alkali metals include sodium, potassium, lithium, cesium, and the alkaline earth metals include magnesium, calcium, and strontium.

Optionally, the pharmaceutically acceptable salt includes a salt formed of a compound of Formula **I** and an acid; optionally, the acid includes an inorganic acid and an organic acid; optionally, the inorganic acid includes hydrochloric acid, hydrobromic acid, hydroiodic acid, sulfuric acid, nitric acid, phosphoric acid, and carbonic acid; and optionally, the organic acid includes formic acid, ascorbic acid, acetic acid, propionic acid, oxalic acid, malonic acid, succinic acid, fumaric acid, maleic acid, lactic acid, malic acid, citric acid, citric acid, tartaric acid, gluconic acid, hydrogen tartaric acid, glucuronic acid, carbonic acid, picric acid, methanesulfonic acid, ethanesulfonic acid, *p*-toluenesulfonic acid, benzoic acid, benzenesulfonic acid, *p*-bromobenzenesulfonic acid, glutamic acid, salicylic acid, and pamoic acid.

According to another aspect of the present disclosure, there is provided a pharmaceutical composition, comprising a therapeutically effective amount of a compound of the following Formula **I:** 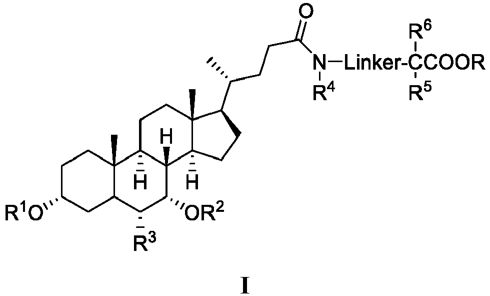 or one or more of pharmaceutically acceptable salts thereof, and optionally a pharmaceutically acceptable carrier,
in Formula **I,**
R¹ and R² each are independently selected from the group consisting of: H, -COR', -CONHR', -CONR'R", or -COOR';
R³ is selected from the group consisting of: H, methyl, or ethyl;
R⁴ is selected from the group consisting of: H, alkyl or substituted alkyl, alkenyl or substituted alkenyl, alkynyl or substituted alkynyl, cycloalkyl or substituted cycloalkyl, aryl or substituted aryl, heteroaryl or substituted heteroaryl, and heterocyclyl or substituted heterocyclyl;
Linker is absent, or is selected from the group consisting of: alkylene or substituted alkylene, alkenylene or substituted alkenylene, alkynylene or substituted alkynylene, cycloalkylene or substituted cycloalkylene, arylene or substituted arylene, heteroarylene or substituted heteroarylene, and heterocyclylene or substituted heterocyclylene;
R⁵ and R⁶ each are independently selected from the group consisting of: H, and alkyl or substituted alkyl; or R⁵ and R⁶ are linked to each other, and form a 3-7 membered ring with 0, 1, or 2 heteroatoms selected from the group consisting of O, S, or N, together with C atoms linked thereto;
R is selected from the group consisting of: alkyl or substituted alkyl, alkenyl or substituted alkenyl, alkynyl or substituted alkynyl, cycloalkyl or substituted cycloalkyl, aryl or substituted aryl, alkylene-aryl, alkylene-substituted aryl, heteroaryl or substituted heteroaryl, and heterocyclyl or substituted heterocyclyl; or
R is selected from the group consisting of: inorganic cations selected from the group consisting of sodium ions, potassium ions, magnesium ions, or calcium ions; or organic cations selected from the group consisting of ammonium, tetramethylammonium, tetraethylammonium, tetrapropylammonium, or tetrabutyl ammonium;
R' and R" each are independently selected from the group consisting of: H, alkyl or substituted alkyl, alkenyl or substituted alkenyl, alkynyl or substituted alkynyl, cycloalkyl or substituted cycloalkyl, aryl or substituted aryl, heteroaryl or substituted heteroaryl, and heterocyclyl or substituted heterocyclyl; and
substituents of the substituted alkyl, the substituted alkylene, the substituted alkenyl, the substituted alkenylene, the substituted alkynyl, the substituted alkynylene, the substituted cycloalkyl, the substituted cycloalkylene, the substituted aryl, the substituted arylene, the alkylene-substituted aryl, the substituted heteroaryl, the substituted heteroarylene, the substituted heterocyclyl, or the substituted heterocyclylene each are independently selected from the group consisting of: halogen, cyano, amino, nitro, hydroxy, alkyl, alkoxyl, and modified alkyl.

Optionally, in the pharmaceutical composition, optionally the "halogen" is selected from the group consisting of: F, Cl, Br, or I;
optionally, "alkyl" in the "alkyl" and "alkoxyl" is C₁-C₂₀ linear or branched alkyl, optionally selected from the group consisting of: methyl, ethyl, n-propyl, isopropyl, n-butyl, isobutyl, tert-butyl, sec-butyl, and n-pentyl;
optionally, "alkylene" in the "alkylene", "alkylene-aryl", and "alkylene-substituted aryl" is C₁-C₁₀ linear or branched alkylene, optionally selected from the group consisting of: methylene, ethylene, n-propylidene, isopropylidene, n-butylidene, isobutylidene, tert-butylidene, sec-butylidene, and n-pentylene;
optionally, the "modified alkyl" is a group obtained by substituting one or more groups selected from the group consisting of -O-, -CO-, -NH₂, -OH, halogen, -CN, and -NO₂ for any carbon atom in the alkyl;
optionally, the "alkenyl" is C₂-C₆ alkenyl;
optionally, the "alkenylene" is C₂-C₆ alkenylene;
optionally, the "alkynyl" is C₂-C₆ alkynyl;
optionally, the "alkynylene" is C₂-C₆ alkynylene;
optionally, the "cycloalkyl" is C₃-C₁₀ monocyclic or bicyclic cycloalkyl;
optionally, the "cycloalkylene" is C₃-C₁₀ monocyclic or bicyclic cycloalkylene;
optionally, "aryl" in the "aryl", the "alkylene-aryl", and the "alkylene-substituted aryl" is 6-10 membered aryl; and is optionally phenyl or naphthyl;
optionally, the "arylene" is 6-10 membered arylene; and may be optionally phenylene or naphthylene;
optionally, the "heteroaryl" is a 5-10 membered heteroaromatic ring containing 1, 2, or 3 heteroatoms selected from the group consisting of N, O, and S;
optionally, the "heteroarylene" is a 5-10 membered heteroarylene ring containing 1, 2, or 3 heteroatoms selected from the group consisting of N, O, and S;
optionally, the "heterocyclyl" is a 3-10 membered nonaromatic heterocyclic ring containing 1, 2, or 3 heteroatoms selected from the group consisting of N, O, and S; and
optionally, the "heterocyclylene" is a 3-10 membered nonaromatic heterocyclylene ring containing 1, 2, or 3 heteroatoms selected from the group consisting of N, O, and S.

Optionally, R' and R" each are independently selected from the group consisting of: H, methyl, ethyl, n-propyl, isopropyl, n-butyl, isobutyl, benzyl, sec-butyl, n-pentyl, 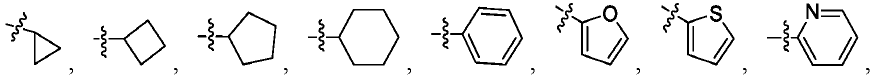 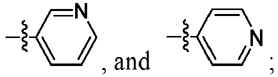
optionally, R¹ and R² each are independently selected from the group consisting of: H, acetyl, propionyl, isopropionyl, butyryl, and isobutyryl;
optionally, R³ is selected from the group consisting of: H, methyl, or ethyl;
optionally, R⁴ is selected from the group consisting of: methyl, ethyl, n-propyl, isopropyl, n-butyl, isobutyl, tert-butyl, sec-butyl, and n-pentyl;
optionally, Linker is absent, or is selected from the group consisting of: -CH₂-, - CH₂CH₂-, -CH₂CH₂CH₂-, cyclopropylidene, 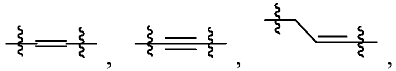 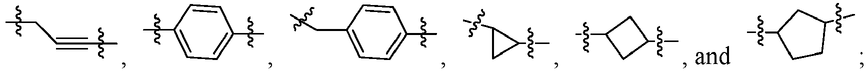
optionally, R⁵ and R⁶ each are independently selected from the group consisting of: methyl, ethyl, n-propyl, isopropyl, n-butyl, isobutyl, tert-butyl, sec-butyl, n-pentyl, or R⁵ and R⁶ are linked to each other, and form, together with the C atoms linked thereto, any group selected from the following formulas: 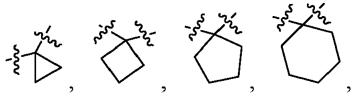 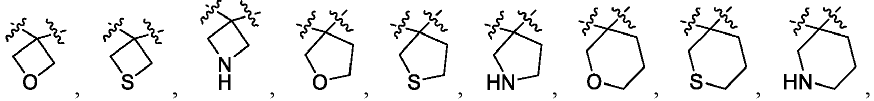 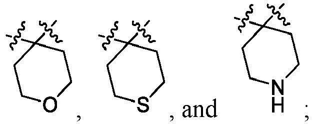 and
optionally, R is selected from the group consisting of: ethyl, propyl, butyl, isopropyl, isobutyl, tert-butyl, sec-butyl, n-pentyl, 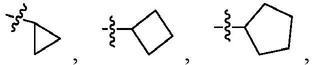 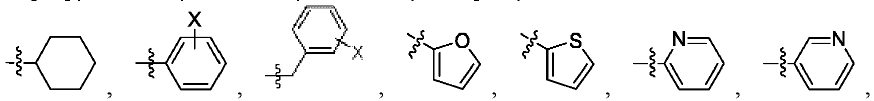 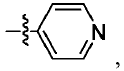 heterocyclyl or substituted heterocyclyl, sodium ions, potassium ions, magnesium ions, calcium ions, ammonium, tetramethylammonium, tetraethylammonium, tetrapropylammonium, or tetrabutylammonium; and Xs each are independently selected from the group consisting of: 0, 1, 2, or 3 groups selected from the group consisting of: F, Cl, Br, I, -NH₂, -OH, -methoxyl, ethoxyl, propoxyl, isopropoxyl, -CN, methyl, ethyl, n-propyl, isopropyl, n-butyl, or isobutyl, or Xs each are independently a group obtained by substituting one or more groups selected from the group consisting of -O-, -CO-, and -NH₂ for any carbon atom on C₅-C₁₀ linear or branched alkyl.

Optionally, in the pharmaceutical composition, the compound of Formula **I** is selected from a compound of the following Formula **II**: 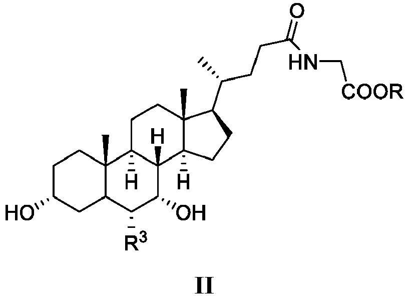 wherein:
R³ and R are as defined above.

Optionally, in the pharmaceutical composition, R³ is selected from the group consisting of: H, methyl, or ethyl; R is selected from the group consisting of: ethyl, n-propyl, isopropyl, n-butyl, isobutyl, 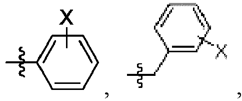 sodium ions, potassium ions, calcium ions, and Xs each are independently selected from the group consisting of: 0, 1, 2, or 3 groups selected from the group consisting of: F, Cl, Br, I, -NH₂, -OH, - methoxyl, ethoxyl, propoxyl, isopropoxyl, -CN, methyl, ethyl, n-propyl, isopropyl, n-butyl, or isobutyl, or Xs each are independently a group obtained by substituting one or more groups selected from the group consisting of -O-, -CO-, and -NH₂ for any carbon atom on C₅-C₁₀ linear or branched alkyl.

Optionally, in the pharmaceutical composition, the compound of the Formula **I** is selected from the following compounds: 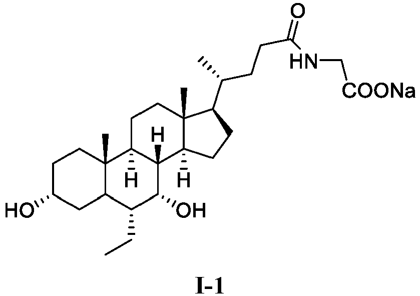 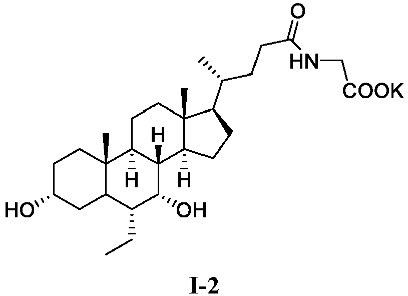 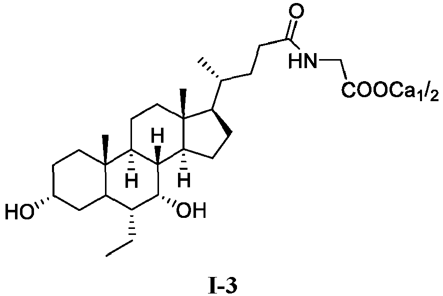 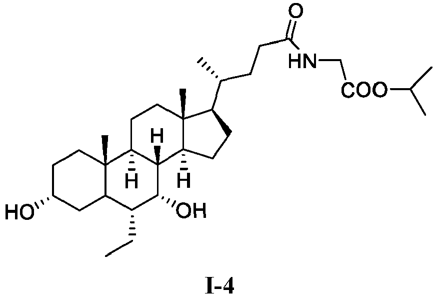 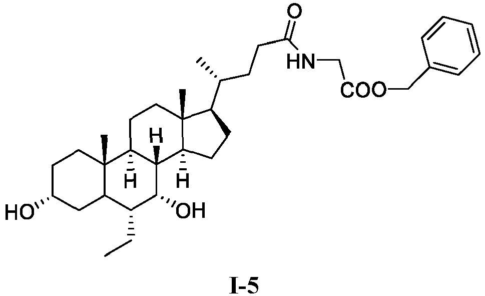 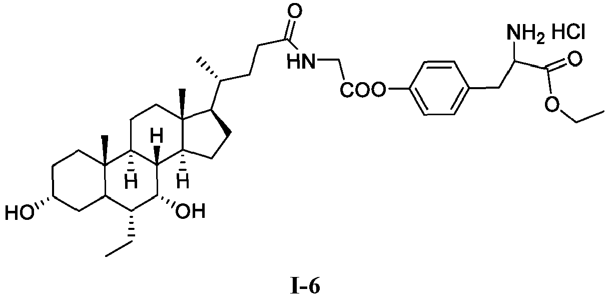 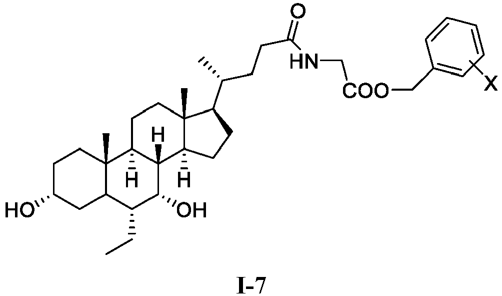
in Formula **I-7,** X is 1, 2, or 3 substituents, each independently selected from the group consisting of F, Cl, Br, I, -CN, -NH₂, -NO₂, or -OH, methyl, ethyl, propyl, isopropyl, methoxyl, ethoxyl, propoxyl, and isopropoxyl; and 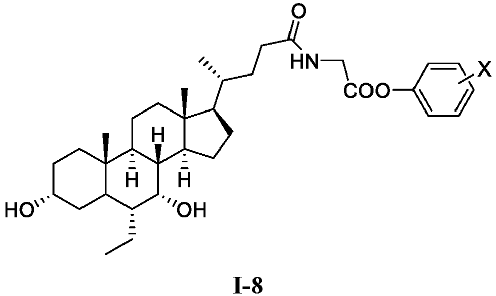
in Formula **I-8,** X is 1, 2, or 3 substituents, each independently selected from the group consisting of F, Cl, Br, I, -CN, -NH₂, -NO₂, or -OH, methyl, ethyl, propyl, isopropyl, methoxyl, ethoxyl, propoxyl, and isopropoxyl.

Optionally, in the pharmaceutical composition, the pharmaceutically acceptable salt includes a salt formed of a compound of Formula **I** and an acid; optionally, the acid includes an inorganic acid and an organic acid; optionally, the inorganic acid includes hydrochloric acid, hydrobromic acid, hydroiodic acid, sulfuric acid, nitric acid, phosphoric acid, and carbonic acid; and optionally, the organic acid includes formic acid, ascorbic acid, acetic acid, propionic acid, oxalic acid, malonic acid, succinic acid, fumaric acid, maleic acid, lactic acid, malic acid, citric acid, citric acid, tartaric acid, gluconic acid, hydrogen tartaric acid, glucuronic acid, carbonic acid, picric acid, methanesulfonic acid, ethanesulfonic acid, *p*-toluenesulfonic acid, benzoic acid, benzenesulfonic acid, *p*-bromobenzenesulfonic acid, glutamic acid, salicylic acid, and pamoic acid.

Optionally, a dosage form of the pharmaceutical composition includes an oral preparation and an injection preparation;
optionally, the oral preparation includes a solid preparation and a liquid preparation;
optionally, the solid preparation includes tablets, powders, granules, and capsules; and
optionally, the liquid preparation includes water or oil suspensions, and syrups.

According to another aspect, there is provided use of the pharmaceutical composition described above, or the compound of Formula **I** or the pharmaceutically acceptable salt thereof as described above in the preparation of a medicament for treating nonalcoholic steatohepatitis.

According to another aspect, there is provided use of the pharmaceutical composition described above, or the compound of Formula **I** or the pharmaceutically acceptable salt thereof as described above in the preparation of a medicament for treating primary biliary cirrhosis.

According to another aspect, there is provided use of the pharmaceutical composition described above, or the compound of Formula **I** or the pharmaceutically acceptable salt thereof as described above in the preparation of a medicament for treating a biliation-associated disease;
optionally, the biliation-associated disease includes portal hypertension, bile acid diarrhea, alcoholic hepatitis, and primary sclerotic cholangitis.

According to another aspect, there is provided a method for treating nonalcoholic steatohepatitis, characterized in administering, to a patient, a therapeutically effective amount of the compound of Formula **I** or the pharmaceutically acceptable salt thereof as described above, or the pharmaceutical composition as described above.

According to another aspect, there is provided a method for treating primary biliary cirrhosis, characterized in administering, to a patient, a therapeutically effective amount of the compound of Formula **I** or the pharmaceutically acceptable salt thereof as described above, or the pharmaceutical composition as described above.

According to another aspect, there is provided a method for treating a biliation-associated disease, characterized in administering, to a patient, a therapeutically effective amount of the compound of Formula **I** or the pharmaceutically acceptable salt thereof as described above, or the pharmaceutical composition as described above;
optionally, the biliation-associated disease includes portal hypertension, bile acid diarrhea, alcoholic hepatitis, and primary sclerotic cholangitis.

### DETAILED DESCRIPTION

The specific embodiments of the present disclosure will be elaborated below. It should be appreciated that the specific embodiments described herein are merely intended to exemplarily illustrate, and not limit the present disclosure.

### Example 1: Preparation of Compounds

### 1. Preparation of Compound 4:

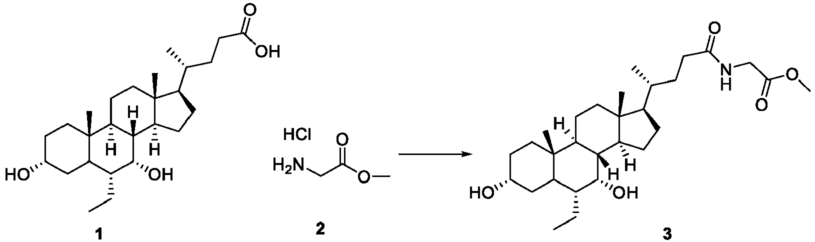

A. Raw Material **1** (0.8 g, 1.90 mmol, 1.0 eq), glycine methyl ester hydrochloride (2,356 mg, 2.85 mmol), EDCI (546 mg, 2.85 mmol), and DMAP (696 mg, 5.70 mmol) were dissolved in DCM (10 mL), and reacted overnight at room temperature under nitrogen protection. The reaction was detected by TLC to be completed. The reactant was rotary-evaporated to remove DCM, and EtOAC was added for dissolution. The solution was washed with IN HCl 5mL × 2 to remove excess DMAP. The organic phase was washed with a saturated salt solution, dried over anhydrous sodium sulfate, filtered, and concentrated to give Compound **3** (900 mg, 96%). ¹H NMR (400 MHz, CDCl₃) δ 5.94(s, 1H), 4.0 (d, *J* = 5.1 Hz, 2H), 3.76 (s, 3H), 3.70(s, 1H), 3.44-3.36 (m, 1H), 2.34-2.27 (m, 1H), 2.18-2.10 (m, 1H), 1.97-0.89 (m, 36H), 0.65 (s, 3H). 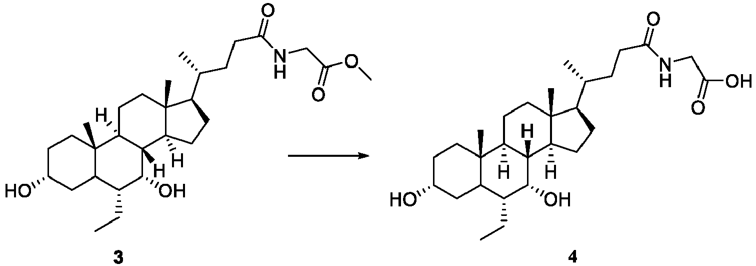
B. Compound **3** (900 mg, 1.83 mmol), and NaOH (111 mg, 2.78 mmol) were added to CH₃OH-H₂O (4 mL, V: V=1:1), and subjected to a reflux reaction for 3h. The reaction was detected by TLC to be completed. The reactant was rotary-evaporated to remove methanol, and extracted by adding EtOAC to remove organic impurities. The aqueous phase was adjusted with IN HCl till the pH was about 5. A white solid was precipitated, and dissolved and extracted by adding EtOAC. The organic phase was washed with a saturated salt solution, dried over anhydrous sodium sulfate, filtered, and concentrated to give Compound **4** (795 mg, 91%). ¹H NMR (400 MHz, DMSO) δ 12.4 (s, 1H), 8.1 (t, *J* =5.9Hz, 1H), 4.3 (s, 1H), 4.05 (d, *J* =4.9Hz, 1H), 3.7 (d, *J* =5.9Hz, 2H), 3.5 (s, 1H), 3.16-3.10 (m, 1H),2.18-2.11 (m, 1H), 2.07-2.01 (m, 1H), 1.97-0.89 (m, 34H), 0.61 (s, 3H); *m*/*z*(ESI) [M-H]⁻=476.4.

### 2. Preparation of Compound I-4:

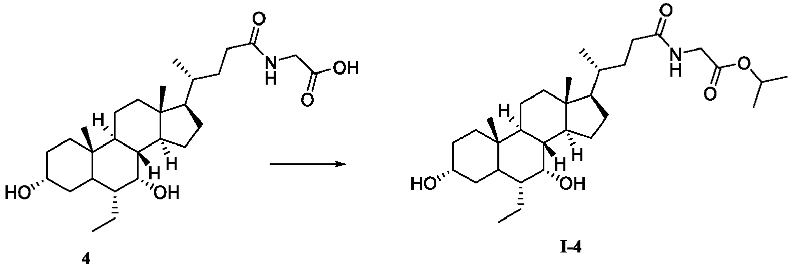

Raw Material **4** (1.3 g, 2.72 mmol), isopropanol (245 mg, 4.08 mmol), EDCI (782 mg, 4.08 mmol), and DMAP (498 mg, 4.08 mmol) were dissolved in DCM (10 mL), and reacted overnight at room temperature under nitrogen protection. The reaction was detected by TLC to be completed. The reactant was rotary-evaporated to remove DCM, and EtOAC was added for dissolution. The solution was washed with IN HCl 10 mL × 2 to remove excess DMAP. The organic phase was washed with a saturated salt solution, dried over anhydrous sodium sulfate, filtered, concentrated, and subjected to column chromatography (DCM:MeOH=20:1) to give Compound **I-4** (653 mg, 46%). ¹H NMR (400 MHz, CDCl₃) δ 5.94 (s, 1H), 5.10-5.04 (m, 1H), 4.0 (d, *J* =5.0 Hz, 2H), 3.7 (s, 1H), 3.44-3.36 (m, 1H), 2.34-2.27 (m, 1H), 2.17-2.07 (m, 1H), 1.97-0.89 (m, 42H), 0.65 (s, 3H).

### 3. Preparation of Compound I-5:

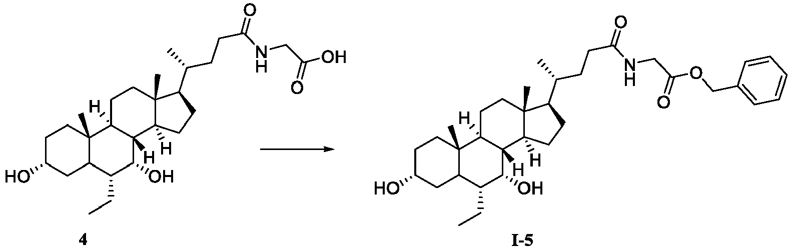

Raw Material **4** (2.27 g, 4.75 mmol), phenylmethanol (770 mg, 7.12 mmol), EDCI (1.36 g, 7.12 mmol), and DMAP (870 mg, 7.12 mmol) were dissolved in DCM (30 mL), and reacted overnight at room temperature under nitrogen protection. The reaction was detected by TLC to be completed. The reactant was rotary-evaporated to remove DCM, and EtOAC was added for dissolution. The solution was washed with IN HCl 10 mL × 2 to remove excess DMAP. The organic phase was washed with a saturated salt solution, dried over anhydrous sodium sulfate, filtered, concentrated, and subjected to column chromatography (DCM:MeOH=20:1) to give Compound **I-5** (1.72 g, 64%). ¹H NMR (400 MHz, CDCl₃) δ 7.39-7.32 (m, 5H), 5.94 (t, *J*=4.8Hz, 1H), 5.18 (s, 2H) ,4.08 (d, *J* =5.2Hz, 2H), 3.7 (s, 1H), 3.44-3.36 (m, 1H), 2.34-2.27 (m, 1H), 2.17-2.07 (m, 1H), 1.97-0.89 (m, 36H), 0.65 (s, 3H); *m*/*z*(ESI) [M+Nₐ]⁺=590.5.

### 4. Preparation of Compound I-6:

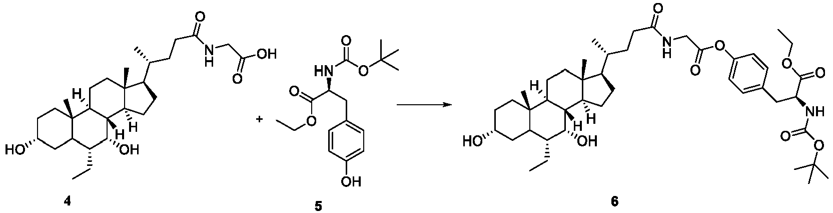

A. Raw Material **4** (2.27 g, 4.75 mmol), Compound **5** (2.20 g, 7.12 mmol), EDCI (1.36 g, 7.12 mmol), and DMAP (870 mg, 7.12 mmol) were dissolved in DCM (30 mL), and reacted overnight at room temperature under nitrogen protection. The reaction was detected by LC-MS to be completed. The reaction was quenched by adding water. The reactant was extracted. The organic phase was washed with a saturated salt solution, dried over anhydrous sodium sulfate, filtered, concentrated, and subjected to column chromatography (DCM:MeOH=20:1) to give Compound **6** (750 mg, 21%). ¹H NMR (400 MHz, CDCl₃) δ 7.15 (d, *J* =8.5Hz, 2H), 7.03 (d, *J* =8.5Hz, 2H), 5.98 (t, *J* =5.2Hz, 1H), 5.0 (d, *J* =8.0Hz, 1H), 4.54 (dd, *J* =6.3Hz, *J* =13.8Hz, 1H), 4.28 (d, *J* =5.2Hz, 2H), 4.15 (t, *J* =7.2Hz, 2H), 3.70 (s,1H), 3.40 (s,1H), 3.13-3.02 (m, 2H),2.37-2.29 (m, 1H), 2.21-2.13 (m, 1H), 1.97-0.89 (m, 48H), 0.65 (s, 3H). 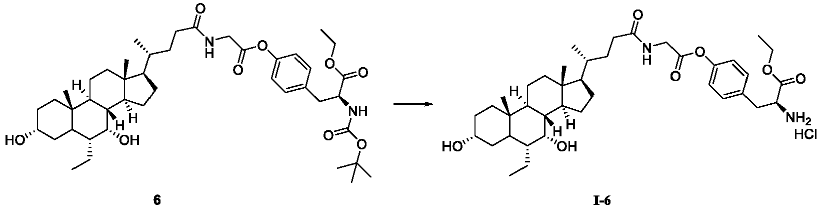
B. Compound **6** (750 mg, 0.98 mmol) was dissolved in DCM (10 mL), into which HCl gas was charged, and the solution was changed from clear to turbid. The reaction was detected by TLC to be completed. The reactant was rotary-evaporated to remove the solvent, and extracted twice with ether, and the reactant was turned into a foamed solid, thereby giving Compound **I-6** (550 mg, 84%). ¹H NMR (400MHz, DMSO) δ 8.67 (d, *J*=4.2Hz, 3H), 8.45 (t, *J*=5.7Hz, 1H), 7.30 (d, *J*=8.5Hz, 2H), 7.07 (d, *J*=8.5Hz, 2H), 4.24 (dd, *J*=6.8Hz, *J*=12.4Hz, 1H), 4.14-4.08 (m, 4H), 3.49 (s, 1H),3.24-3.03 (m, 3H), 2.22-2.16 (m, 1H),2.12-2.05 (m, 1H), 1.97-0.89 (m, 39H), 0.59(s, 3H); *m*/*z*(ESI) [M-HCl+H]⁺=669.5.

### 5. Preparation of Compound I-1:

Compound **3** was hydrolyzed with NaOH, or Compound **4** was treated with an aqueous NaOH solution to give compound **I**-1.

### Test Example 1: Pharmacokinetic Test

1. Formulation of preparation and dosing: An appropriate amount of the test sample was precisely weighed, and mixed with an appropriate volume of a solvent to obtain a clear solution or a homogeneous suspension. The preparation was administered to animals within 4 hours after formulation. The dosage formulation would be administered by oral gavage in accordance with facility standard operating procedures. The dosage volume would be determined by the body weights of the animals collected on the morning of administering.
2. Blood collection: Blood (approx. 0.2 ml per point in time) would be acupunctured each time from the jugular vein of every animal into a polypropylene tube. All blood samples would be transferred to pre-cooled commercial EDTA-K2 test tubes, and kept on wet ice until they were centrifuged.
3. Plasma processing: Blood samples would be centrifuged (3200 rpm, 10 min) at approximately 4°C. Plasma was collected separately and transferred to pre-labeled PP tubes in wet ice at each point in time, and then immediately precipitated by ACN (6 IS) (the ratio of plasma: ACN was 1:4). Centrifugation (10 min, 12,000 rpm) was carried out again to obtain a supernatant. The supernatant was quickly frozen on dry ice and kept at -70 ± 10°C before LC/MS/MS analysis.
4. Liver processing: Liver tissues were collected at each point in time, washed twice with pre-cooled deionized water, and dried with filter paper. Liver tissues were immediately homogenized with 10-times volume of a methanol-water solution (1:2, v/v). After homogenization, a portion of liver tissue homogenate (e.g., 200 uL of homogenate) was immediately measured and delivered to an analyst. After precipitated on wet ice, the samples were centrifuged, and a supernatant was collected and stored in a refrigerator at -70 ± 10°C before LC-MS/MS analysis. From the remaining liver homogenate, 800 uL of the homogenate was measured as a spare.

Compounds **(I-4, I-5, I-6** and OCA, 0.0576 mmol/kg for each) were administered to rats by gavage, respectively. Rat plasma and liver samples were collected at points in time of 0.25, 0.5, 1, 2, 4, 8, 12, and 24 h, respectively, and tested by LC-MS/MS according to the above-mentioned method to determine the concentrations of the *in vivo* active metabolites of the above-mentioned compounds in plasma and liver (see Tables 1-4).

After Compound **I-4** was administered to rats by gavage, the *in vivo* active compounds in the rats were Compound **4.** The concentrations of Compound **4** in plasma and liver were as shown in Table 1.

**Table 1**

| | **Compound 4** | |
|---|---|---|
| PK Parameters | Plasma | Liver |
| Rsq_adj | 0.930 | 0.868 |
| No. points used for T_{1/2} | 5.00 | 5.00 |
| Cₘₐₓ (ng/mL of ng/g) | 889 | 10494 |
| Tₘₐₓ (h) | 1.00 | 1.00 |
| T_{1/2} (h) | 4.79 | 5.61 |
| Tₗₐₛₜ (h) | 24.0 | 24.0 |
| AUC₀₋ₗₐₛₜ (ng.h/mL or ng.h/g) | 4845 | 105664 |
| AUC_{0-inf} (ng.h/mL or ng.h/g) | 4997 | 111955 |
| MRT₀₋ₗₐₛₜ (h) | 5.91 | 6.80 |
| MRT_{0-inf} (h) | 6.67 | 8.22 |
| AUC_{Extra} (%) | 3.04 | 5.62 |
| AUMC_{Extra} (%) | 14.1 | 21.9 |
| AUC Ratio | 21.8 | |

| | | |
|---|---|---|
| Total AUC in plasma and liver=110506, AUC Ratio=liver/plasma=105664/4845=21.8. | | |

After Compound **I-5** was administered to rats by gavage, the *in vivo* active compounds in the rats were Compound **4.** The concentrations of Compound **4** in plasma and liver were as shown in Table 2.

**Table 2**

| | **Compound 4** | |
|---|---|---|
| PK Parameters | Plasma | Liver |
| Rsq_adj | 0.961 | 0.994 |
| No. points used for T_{1/2} | 7.00 | 4.00 |
| Cₘₐₓ (ng/mL of ng/g) | 2047 | 31790 |
| Tₘₐₓ (h) | 0.250 | 0.250 |
| T_{1/2} (h) | 4.24 | 4.58 |
| Tₗₐₛₜ (h) | 24.0 | 24.0 |
| AUC₀₋ₗₐₛₜ (ng.h/mL or ng.h/g) | 7634 | 142808 |
| AUC_{0-inf} (ng.h/mL or ng.h/g) | 7831 | 147182 |
| MRT₀₋ₗₐₛₜ (h) | 5.19 | 6.31 |
| MRT_{0-inf} (h) | 5.81 | 7.03 |
| AUC_{Extra} (%) | 2.51 | 2.97 |
| AUMC_{Extra} (%) | 13.0 | 12.9 |
| AUC Ratio | 18.7 | |

| | | |
|---|---|---|
| Total AUC in plasma and liver=150442, AUC Ratio=liver/plasma=142808/7634=18.7. | | |

After Compound **I-6** was administered to rats by gavage, the *in vivo* active compounds in the rats were Compound **4.** The concentrations of Compound **4** in plasma and liver were as shown in Table 3.

**Table 3**

| | **Compound 4** | |
|---|---|---|
| PK Parameters | Plasma | Liver |
| Rsq_adj | 0.747 | 0.754 |
| No. points used for T_{1/2} | 4.00 | 4.00 |
| Cₘₐₓ (ng/mL of ng/g) | 775 | 18040 |
| Tₘₐₓ (h) | 2.00 | 2.00 |
| T_{1/2} (h) | 6.04 | 6.34 |
| Tₗₐₛₜ (h) | 24.0 | 24.0 |
| AUC₀₋ₗₐₛₜ (ng.h/mL or ng.h/g) | 3858 | 83872 |
| AUC_{0-inf} (ng.h/mL or ng.h/g) | 4131 | 90846 |
| MRT₀₋ₗₐₛₜ (h) | 5.67 | 6.02 |
| MRT_{0-inf} (h) | 7.46 | 8.10 |
| AUC_{Extra} (%) | 6.62 | 7.68 |
| AUMC_{Extra} (%) | 29.0 | 31.4 |
| AUC Ratio | 21.7 | |

| | | |
|---|---|---|
| Total AUC in plasma and liver=87730, AUC Ratio=liver/plasma=83872/3858=21.7. | | |

After the obeticholic acid was administered to rats by gavage, the *in vivo* active compounds in the rats were obeticholic acid (OCA) and Compound **4.** Their concentrations in plasma and liver were as shown in Table 4.

**Table 4**

| | OCA | | **Compound 4** | |
|---|---|---|---|---|
| PK Parameters | Plasma | Liver | Plasma | Liver |
| Rsq_adj | 0.287 | 0.629 | 0.963 | 0.998 |
| No. points used for T_{1/2} | 6.00 | 6.00 | 4.00 | 3.00 |
| Cₘₐₓ (ng/mL) | 24333 | 17930 | 35.6 | 2859 |
| Tₘₐₓ (h) | 0.500 | 0.500 | 1.00 | 1.00 |
| T_{1/2} (h) | 4.35 | 3.33 | 28.7 | 14.5 |
| Tₗₐₛₜ (h) | 24.0 | 24.0 | 24.0 | 24.0 |
| AUC₀₋ₗₐₛₜ (ng.h/mL) | 48093 | 31894 | 263 | 11136 |
| AUC_{0-inf} (ng.h/mL) | 50289 | 32264 | 575 | 15344 |
| AUC Ratio | 0.663 | | 42.3 | |

| | | | | |
|---|---|---|---|---|
| Total AUC in plasma and liver AUC=91386; AUC Ratio=liver/plasma=43 03 0/483 56=0. 89. | | | | |

After Compound **4** was administered to rats by gavage, the *in vivo* active compounds in the rats were Compound **4.** The concentrations of Compound **4** in plasma and liver were as shown in Table 5.

**Table 5**

| | **Compound 4** | |
|---|---|---|
| PK Parameters | Plasma | Liver |
| Rsq_adj | 0.997 | 0.994 |
| No. points used for T_{1/2} | 3.00 | 3.00 |
| Cₘₐₓ (ng/mL of ng/g) | 788 | 16830 |
| Tₘₐₓ (h) | 4.00 | 4.00 |
| T_{1/2} (h) | 4.79 | 4.30 |
| Tₗₐₛₜ (h) | 24.0 | 24.0 |
| AUC₀₋ₗₐₛₜ (ng.h/mL or ng.h/g) | 7309 | 107187 |
| AUC_{0-inf} (ng.h/mL or ng.h/g) | 7463 | 110040 |
| MRT₀₋ₗₐₛₜ (h) | 5.56 | 6.25 |
| MRT_{0-inf} (h) | 6.10 | 6.77 |
| AUC_{Extra} (%) | 2.14 | 2.19 |
| AUMC_{Extra} (%) | 10.8 | 9.78 |
| AUC Ratio | 14.7 | |

| | | |
|---|---|---|
| Total AUC in plasma and liver AUC=117503, AUC Ratio=liver/plasma=14.7. | | |

From the data listed in Tables 1, 2, 3 and 4, it could be seen that after gavage administration of the compound OCA to rats, lots of drug OCA (a sum of AUC of OCA in plasma + AUC of OCA-Gly was 48360) circulated in the blood, which might lead to side effects on skin caused by OCA, and the total effective drug amount (a sum of AUC of OCA in liver + AUC of OCA-Gly was 43030) in the liver was reduced accordingly. However, after Compounds **I-4**, **I-5** and **I-6** of the present disclosure were administered to rats by gavage in the same molar dosage respectively, the total effective drug amount in the liver was significantly increased. In particular, after administration of Compound **1-5,** the AUC of the effective drug in the liver reached 142808, which was 3.3 times that of the effective drug in the liver in case of direct administration of OCA, but the AUC of the effective drug in the blood decreased from 48360 in case of direct administration of OCA to 7634 by 84%. After obeticholic acid-glycine conjugate **4** and benzyl ester **I-5** thereof were administered to rats by gavage in the same molar dosage, the comparison showed that the AUC ratio of the obeticholic acid-glycine conjugate **4** generated by the benzyl ester **I-5** in liver/plasma reached 18.7, while the corresponding AUC ratio in case of direct administration of the obeticholic acid-glycine conjugate **4** was only 14.7. Enrichment of esterase in the liver might be one of the reasons why the benzyl ester **I-5** exhibited a higher liver-targeting characteristic. The total AUC, i.e., 150442 of the Compound **4** generated by the benzyl ester **I-5** in the liver and plasma was also significantly increased by 28% in comparison to the total AUC, i.e., 117503 of the Compound **4** when directly administered by gavage to rats.

Therefore, when the compounds of the present disclosure are used to treat the corresponding liver diseases, it is expected to significantly reduce the side effects on skin, while improving the efficacy of treating the liver diseases, and the dosage required to achieve the same efficacy is significantly reduced.

## Claims

1. A compound of Formula **I:** 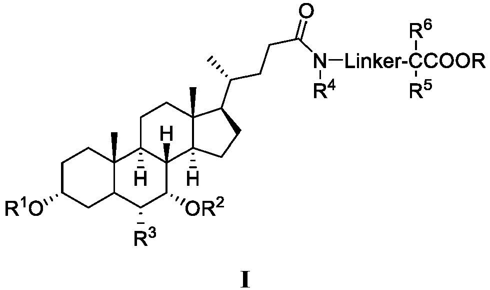 or a pharmaceutically acceptable salt thereof, wherein:
R¹ and R² each are independently selected from the group consisting of: H, -COR', -CONHR', -CONR'R", or -COOR';
R³ is selected from the group consisting of: H, methyl, or ethyl;
R⁴ is selected from the group consisting of: H, alkyl or substituted alkyl, alkenyl or substituted alkenyl, alkynyl or substituted alkynyl, cycloalkyl or substituted cycloalkyl, aryl or substituted aryl, heteroaryl or substituted heteroaryl, and heterocyclyl or substituted heterocyclyl;
Linker is absent, or is selected from the group consisting of: alkylene or substituted alkylene, alkenylene or substituted alkenylene, alkynylene or substituted alkynylene, cycloalkylene or substituted cycloalkylene, arylene or substituted arylene, heteroarylene or substituted heteroarylene, and heterocyclylene or substituted heterocyclylene;
R⁵ and R⁶ each are independently selected from the group consisting of: H, and alkyl or substituted alkyl; or R⁵ and R⁶ are linked to each other, and form a 3-7 membered ring with 0, 1, or 2 heteroatoms selected from the group consisting of O, S, or N, together with C atoms linked thereto;
R is selected from the group consisting of: alkyl or substituted alkyl, alkenyl or substituted alkenyl, alkynyl or substituted alkynyl, cycloalkyl or substituted cycloalkyl, aryl or substituted aryl, alkylene-aryl, alkylene-substituted aryl, heteroaryl or substituted heteroaryl, and heterocyclyl or substituted heterocyclyl; or
R is selected from the group consisting of: inorganic cations selected from the group consisting of sodium ions, potassium ions, magnesium ions, or calcium ions; or organic cations selected from the group consisting of ammonium, tetramethylammonium, tetraethylammonium, tetrapropylammonium, or tetrabutyl ammonium;
R' and R" each are independently selected from the group consisting of: H, alkyl or substituted alkyl, alkenyl or substituted alkenyl, alkynyl or substituted alkynyl, cycloalkyl or substituted cycloalkyl, aryl or substituted aryl, heteroaryl or substituted heteroaryl, and heterocyclyl or substituted heterocyclyl; and
substituents of the substituted alkyl, the substituted alkylene, the substituted alkenyl, the substituted alkenylene, the substituted alkynyl, the substituted alkynylene, the substituted cycloalkyl, the substituted cycloalkylene, the substituted aryl, the substituted arylene, the alkylene-substituted aryl, the substituted heteroaryl, the substituted heteroarylene, the substituted heterocyclyl, or the substituted heterocyclylene each are independently selected from the group consisting of: halogen, cyano, amino, nitro, hydroxy, alkyl, alkoxyl, and modified alkyl.

2. The compound of Formula **I** or the pharmaceutically acceptable salt thereof according to claim 1, wherein the compound of Formula **I** is not a compound: 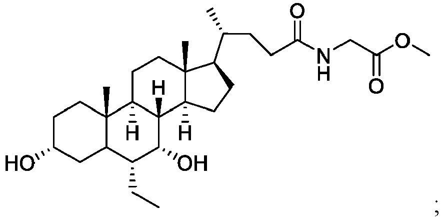 optionally, R in the compound of Formula **I** is not methyl.

3. The compound of Formula **I** or the pharmaceutically acceptable salt thereof according to claim 1 or 2, **characterized in that**:
optionally, the "halogen" is selected from the group consisting of: F, Cl, Br, or I;
optionally, "alkyl" in the "alkyl" and "alkoxyl" is C₁-C₂₀ linear or branched alkyl, optionally selected from the group consisting of: methyl, ethyl, n-propyl, isopropyl, n-butyl, isobutyl, tert-butyl, sec-butyl, and n-pentyl;
optionally, "alkylene" in the "alkylene", "alkylene-aryl", and "alkylene-substituted aryl" is C₁-C₁₀ linear or branched alkylene, optionally selected from the group consisting of: methylene, ethylene, n-propylidene, isopropylidene, n-butylidene, isobutylidene, tert-butylidene, sec-butylidene, and n-pentylene;
optionally, the "modified alkyl" is a group obtained by substituting one or more groups selected from the group consisting of -O-, -CO-, -NH₂, -OH, halogen, -CN, and -NO₂ for any carbon atom in the alkyl;
optionally, the "alkenyl" is C₂-C₆ alkenyl;
optionally, the "alkenylene" is C₂-C₆ alkenylene;
optionally, the "alkynyl" is C₂-C₆ alkynyl;
optionally, the "alkynylene" is C₂-C₆ alkynylene;
optionally, the "cycloalkyl" is C₃-C₁₀ monocyclic or bicyclic cycloalkyl;
optionally, the "cycloalkylene" is C₃-C₁₀ monocyclic or bicyclic cycloalkylene;
optionally, "aryl" in the "aryl", the "alkylene-aryl", and the "alkylene-substituted aryl" is 6-10 membered aryl; and is optionally phenyl or naphthyl;
optionally, the "arylene" is 6-10 membered arylene; and may be optionally phenylene or naphthylene;
optionally, the "heteroaryl" is a 5-10 membered heteroaromatic ring containing 1, 2, or 3 heteroatoms selected from the group consisting of N, O, and S;
optionally, the "heteroarylene" is a 5-10 membered heteroarylene ring containing 1, 2, or 3 heteroatoms selected from the group consisting of N, O, and S;
optionally, the "heterocyclyl" is a 3-10 membered nonaromatic heterocyclic ring containing 1, 2, or 3 heteroatoms selected from the group consisting of N, O, and S; and
optionally, the "heterocyclylene" is a 3-10 membered nonaromatic heterocyclylene ring containing 1, 2, or 3 heteroatoms selected from the group consisting of N, O, and S.

4. The compound of Formula **I** or the pharmaceutically acceptable salt thereof according to any one of claims 1 to 3, **characterized in that**:
optionally, R' and R" each are independently selected from the group consisting of: H, methyl, ethyl, n-propyl, isopropyl, n-butyl, isobutyl, benzyl, sec-butyl, n-pentyl, 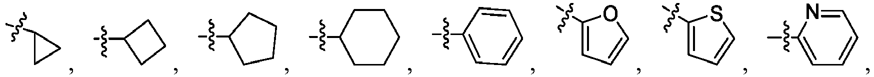 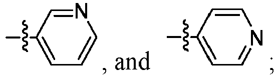
optionally, R¹ and R² each are independently selected from the group consisting of: H, acetyl, propionyl, isopropionyl, butyryl, and isobutyryl;
optionally, R³ is selected from the group consisting of: H, methyl, or ethyl;
optionally, R⁴ is selected from the group consisting of: methyl, ethyl, n-propyl, isopropyl, n-butyl, isobutyl, tert-butyl, sec-butyl, and n-pentyl;
optionally, Linker is absent, or is selected from the group consisting of: -CH₂-, - CH₂CH₂-, -CH₂CH₂CH₂-, cyclopropylidene, 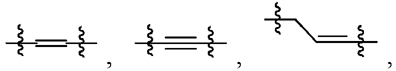 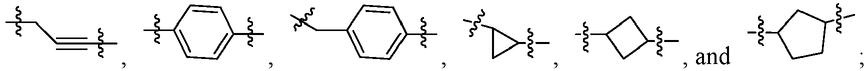
optionally, R⁵ and R⁶ each are independently selected from the group consisting of: methyl, ethyl, n-propyl, isopropyl, n-butyl, isobutyl, tert-butyl, sec-butyl, n-pentyl, or R⁵ and R⁶ are linked to each other, and form, together with the C atoms linked thereto, any group selected from the following formulas: 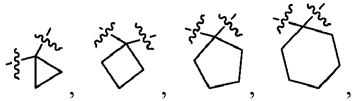 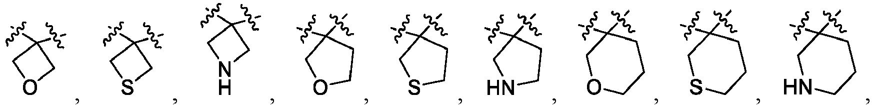 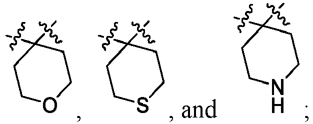 and
optionally, R is selected from the group consisting of: ethyl, propyl, butyl, isopropyl, isobutyl, tert-butyl, sec-butyl, n-pentyl, 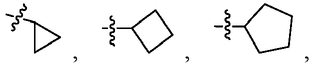 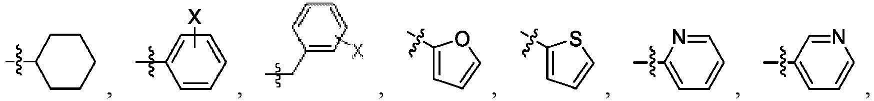 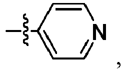 heterocyclyl or substituted heterocyclyl, sodium ions, potassium ions, magnesium ions, calcium ions, ammonium, tetramethylammonium, tetraethylammonium, tetrapropylammonium, or tetrabutylammonium; and Xs each are independently selected from the group consisting of: 0, 1, 2, or 3 groups selected from the group consisting of: F, Cl, Br, I, -NH₂, -OH, -methoxyl, ethoxyl, propoxyl, isopropoxyl, -CN, methyl, ethyl, n-propyl, isopropyl, n-butyl, or isobutyl, or Xs each are independently a group obtained by substituting one or more groups selected from the group consisting of -O-, -CO-, and -NH₂ for any carbon atom on C₅-C₁₀ linear or branched alkyl.

5. The compound of Formula **I** or the pharmaceutically acceptable salt thereof according to any one of claims 1 to 4, **characterized in that** the compound of Formula **I** is selected from a compound of the following Formula **II**: 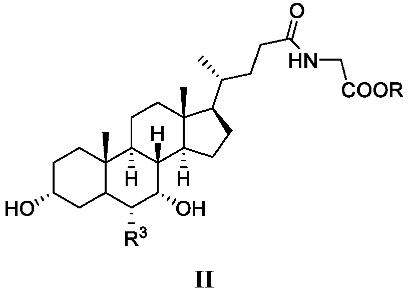 wherein:
R³ and R are as defined in any one of claims 1 to 3.

6. The compound of Formula **I** or the pharmaceutically acceptable salt thereof according to claim 5, **characterized in that** R³ is selected from the group consisting of: H, methyl, or ethyl; R is selected from the group consisting of: ethyl, n-propyl, isopropyl, n-butyl, isobutyl, 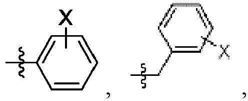 sodium ions, potassium ions, calcium ions, and Xs each are independently selected from the group consisting of: 0, 1, 2, or 3 groups selected from the group consisting of: F, Cl, Br, I, -NH₂, -OH, - methoxyl, ethoxyl, propoxyl, isopropoxyl, -CN, methyl, ethyl, n-propyl, isopropyl, n-butyl, or isobutyl, or Xs each are independently a group obtained by substituting one or more groups selected from the group consisting of -O-, -CO-, and -NH₂ for any carbon atom on C₅-C₁₀ linear or branched alkyl.

7. The compound of Formula **I** or the pharmaceutically acceptable salt thereof according to any one of claims 1 to 6, **characterized in** being selected from the following compounds: 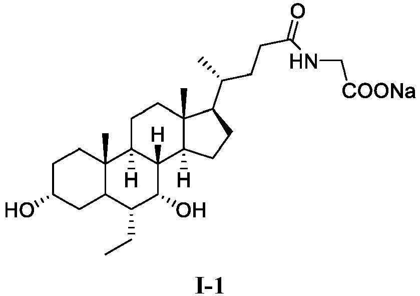 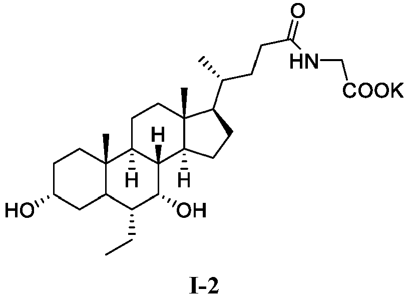 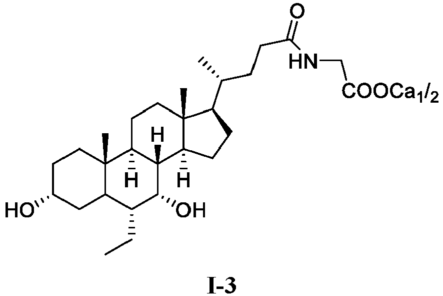 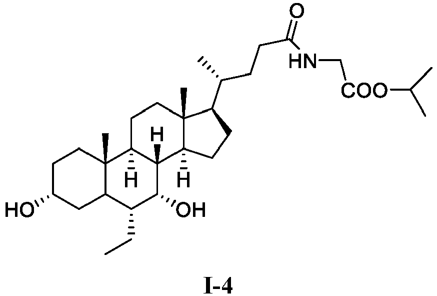 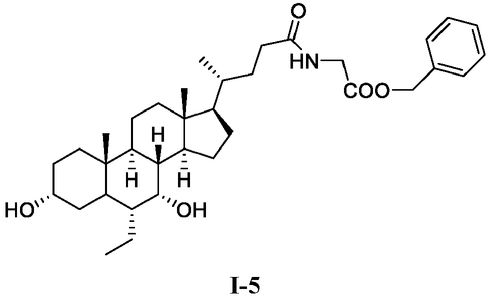 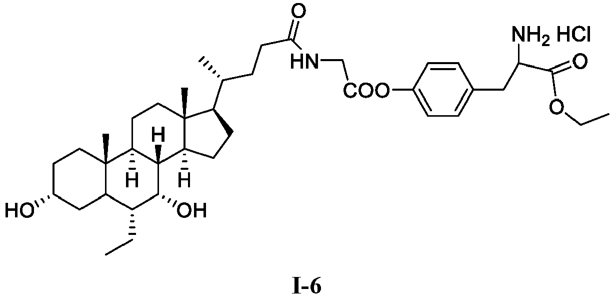 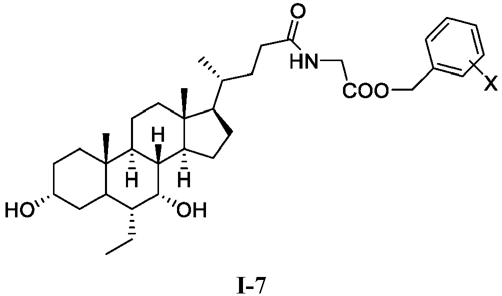
in Formula **I-7,** X is 1, 2, or 3 substituents, each independently selected from the group consisting of F, Cl, Br, I, -CN, -NH₂, -NO₂, or -OH, methyl, ethyl, propyl, isopropyl, methoxyl, ethoxyl, propoxyl, and isopropoxyl; and 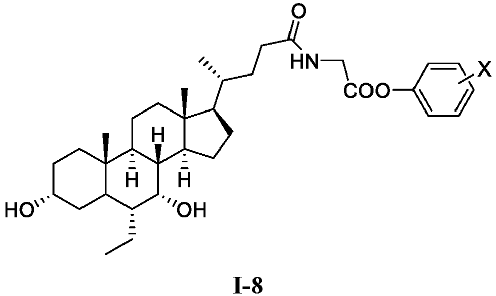
in Formula **I-8,** X is 1, 2, or 3 substituents, each independently selected from the group consisting of F, Cl, Br, I, -CN, -NH₂, -NO₂, or -OH, methyl, ethyl, propyl, isopropyl, methoxyl, ethoxyl, propoxyl, and isopropoxyl.

8. The compound of Formula **I** or the pharmaceutically acceptable salt thereof according to any one of claims 1 to 7, **characterized in that**, the pharmaceutically acceptable salt includes a salt formed of a compound of Formula **I** and an acid; optionally, the acid includes an inorganic acid and an organic acid; optionally, the inorganic acid includes hydrochloric acid, hydrobromic acid, hydroiodic acid, sulfuric acid, nitric acid, phosphoric acid, and carbonic acid; and optionally, the organic acid includes formic acid, ascorbic acid, acetic acid, propionic acid, oxalic acid, malonic acid, succinic acid, fumaric acid, maleic acid, lactic acid, malic acid, citric acid, citric acid, tartaric acid, gluconic acid, hydrogen tartaric acid, glucuronic acid, carbonic acid, picric acid, methanesulfonic acid, ethanesulfonic acid, *p*-toluenesulfonic acid, benzoic acid, benzenesulfonic acid, *p*-bromobenzenesulfonic acid, glutamic acid, salicylic acid, and pamoic acid.

9. A pharmaceutical composition, **characterized in** comprising a therapeutically effective amount of a compound of the following Formula **I:** 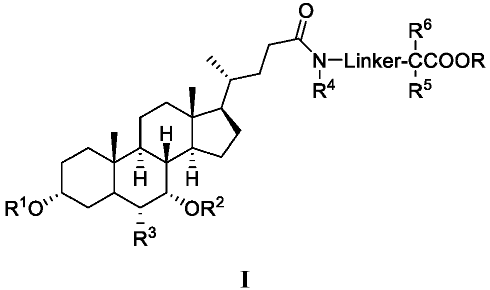
or one or more of pharmaceutically acceptable salts thereof, and optionally a pharmaceutically acceptable carrier,
in Formula **I,**
R¹ and R² each are independently selected from the group consisting of: H, -COR', -CONHR', -CONR'R", or -COOR';
R³ is selected from the group consisting of: H, methyl, or ethyl;
R⁴ is selected from the group consisting of: H, alkyl or substituted alkyl, alkenyl or substituted alkenyl, alkynyl or substituted alkynyl, cycloalkyl or substituted cycloalkyl, aryl or substituted aryl, heteroaryl or substituted heteroaryl, and heterocyclyl or substituted heterocyclyl;
Linker is absent, or is selected from the group consisting of: alkylene or substituted alkylene, alkenylene or substituted alkenylene, alkynylene or substituted alkynylene, cycloalkylene or substituted cycloalkylene, arylene or substituted arylene, heteroarylene or substituted heteroarylene, and heterocyclylene or substituted heterocyclylene;
R⁵ and R⁶ each are independently selected from the group consisting of: H, and alkyl or substituted alkyl; or R⁵ and R⁶ are linked to each other, and form a 3-7 membered ring with 0, 1, or 2 heteroatoms selected from the group consisting of O, S, or N, together with C atoms linked thereto;
R is selected from the group consisting of: alkyl or substituted alkyl, alkenyl or substituted alkenyl, alkynyl or substituted alkynyl, cycloalkyl or substituted cycloalkyl, aryl or substituted aryl, alkylene-aryl, alkylene-substituted aryl, heteroaryl or substituted heteroaryl, and heterocyclyl or substituted heterocyclyl; or
R is selected from the group consisting of: inorganic cations selected from the group consisting of sodium ions, potassium ions, magnesium ions, or calcium ions; or organic cations selected from the group consisting of ammonium, tetramethylammonium, tetraethylammonium, tetrapropylammonium, or tetrabutyl ammonium;
R' and R" each are independently selected from the group consisting of: H, alkyl or substituted alkyl, alkenyl or substituted alkenyl, alkynyl or substituted alkynyl, cycloalkyl or substituted cycloalkyl, aryl or substituted aryl, heteroaryl or substituted heteroaryl, and heterocyclyl or substituted heterocyclyl; and
substituents of the substituted alkyl, the substituted alkylene, the substituted alkenyl, the substituted alkenylene, the substituted alkynyl, the substituted alkynylene, the substituted cycloalkyl, the substituted cycloalkylene, the substituted aryl, the substituted arylene, the alkylene-substituted aryl, the substituted heteroaryl, the substituted heteroarylene, the substituted heterocyclyl, or the substituted heterocyclylene each are independently selected from the group consisting of: halogen, cyano, amino, nitro, hydroxy, alkyl, alkoxyl, and modified alkyl.

10. The pharmaceutical composition according to claim 9, **characterized in that**:
optionally, the "halogen" is selected from the group consisting of: F, Cl, Br, or I;
optionally, "alkyl" in the "alkyl" and "alkoxyl" is C₁-C₂₀ linear or branched alkyl, optionally selected from the group consisting of: methyl, ethyl, n-propyl, isopropyl, n-butyl, isobutyl, tert-butyl, sec-butyl, and n-pentyl;
optionally, "alkylene" in the "alkylene", "alkylene-aryl", and "alkylene-substituted aryl" is C₁-C₁₀ linear or branched alkylene, optionally selected from the group consisting of: methylene, ethylene, n-propylidene, isopropylidene, n-butylidene, isobutylidene, tert-butylidene, sec-butylidene, and n-pentylene;
optionally, the "modified alkyl" is a group obtained by substituting one or more groups selected from the group consisting of -O-, -CO-, -NH₂, -OH, halogen, -CN, and -NO₂ for any carbon atom in the alkyl;
optionally, the "alkenyl" is C₂-C₆ alkenyl;
optionally, the "alkenylene" is C₂-C₆ alkenylene;
optionally, the "alkynyl" is C₂-C₆ alkynyl;
optionally, the "alkynylene" is C₂-C₆ alkynylene;
optionally, the "cycloalkyl" is C₃-C₁₀ monocyclic or bicyclic cycloalkyl;
optionally, the "cycloalkylene" is C₃-C₁₀ monocyclic or bicyclic cycloalkylene;
optionally, "aryl" in the "aryl", the "alkylene-aryl", and the "alkylene-substituted aryl" is 6-10 membered aryl; and is optionally phenyl or naphthyl;
optionally, the "arylene" is 6-10 membered arylene; and may be optionally phenylene or naphthylene;
optionally, the "heteroaryl" is a 5-10 membered heteroaromatic ring containing 1, 2, or 3 heteroatoms selected from the group consisting of N, O, and S;
optionally, the "heteroarylene" is a 5-10 membered heteroarylene ring containing 1, 2, or 3 heteroatoms selected from the group consisting of N, O, and S;
optionally, the "heterocyclyl" is a 3-10 membered nonaromatic heterocyclic ring containing 1, 2, or 3 heteroatoms selected from the group consisting of N, O, and S; and
optionally, the "heterocyclylene" is a 3-10 membered nonaromatic heterocyclylene ring containing 1, 2, or 3 heteroatoms selected from the group consisting of N, O, and S.

11. The pharmaceutical composition according to claim 9 or 10, **characterized in that**:
optionally, R' and R" each are independently selected from the group consisting of: H, methyl, ethyl, n-propyl, isopropyl, n-butyl, isobutyl, benzyl, sec-butyl, n-pentyl, 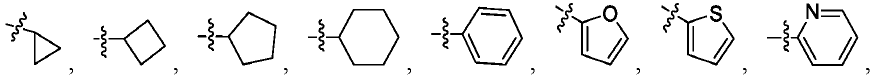 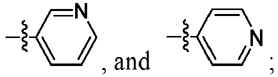
optionally, R¹ and R² each are independently selected from the group consisting of: H, acetyl, propionyl, isopropionyl, butyryl, and isobutyryl;
optionally, R³ is selected from the group consisting of: H, methyl, or ethyl;
optionally, R⁴ is selected from the group consisting of: methyl, ethyl, n-propyl, isopropyl, n-butyl, isobutyl, tert-butyl, sec-butyl, and n-pentyl;
optionally, Linker is absent, or is selected from the group consisting of: -CH₂-, - CH₂CH₂-, -CH₂CH₂CH₂-, cyclopropylidene, 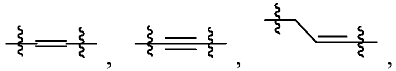 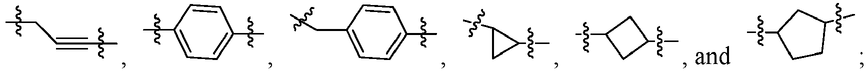
optionally, R⁵ and R⁶ each are independently selected from the group consisting of: methyl, ethyl, n-propyl, isopropyl, n-butyl, isobutyl, tert-butyl, sec-butyl, n-pentyl, or R⁵ and R⁶ are linked to each other, and form, together with the C atoms linked thereto, any group selected from the following formulas: 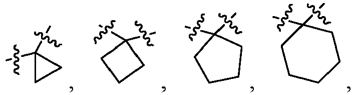 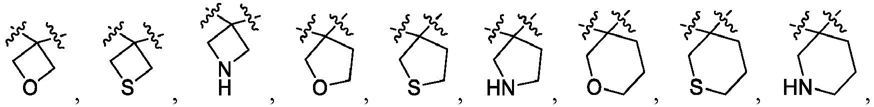 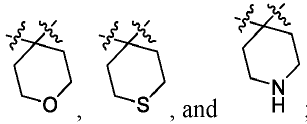 , and
optionally, R is selected from the group consisting of: ethyl, propyl, butyl, isopropyl, isobutyl, tert-butyl, sec-butyl, n-pentyl, 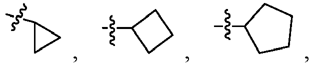 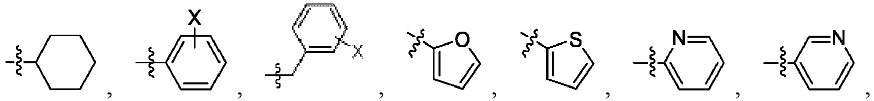 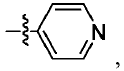 heterocyclyl or substituted heterocyclyl, sodium ions, potassium ions, magnesium ions, calcium ions, ammonium, tetramethylammonium, tetraethylammonium, tetrapropylammonium, or tetrabutylammonium; and Xs each are independently selected from the group consisting of: 0, 1, 2, or 3 groups selected from the group consisting of: F, Cl, Br, I, -NH₂, -OH, -methoxyl, ethoxyl, propoxyl, isopropoxyl, -CN, methyl, ethyl, n-propyl, isopropyl, n-butyl, or isobutyl, or Xs each are independently a group obtained by substituting one or more groups selected from the group consisting of -O-, -CO-, and -NH₂ for any carbon atom on C₅-C₁₀ linear or branched alkyl.

12. The pharmaceutical composition according to any one of claims 9 to 11, **characterized in that** the compound of Formula **I** is selected from a compound of the following Formula **II:** 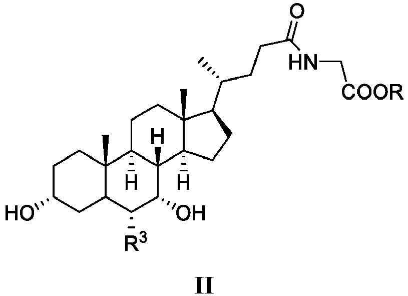 wherein:
R³ and R are as defined in any one of claims 9 to 11.

13. The pharmacetucial composition according to claim 12, **characterized in that** R³ is selected from the group consisting of: H, methyl, or ethyl; R is selected from the group consisting of: ethyl, n-propyl, isopropyl, n-butyl, isobutyl, 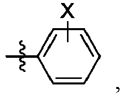 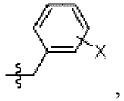 sodium ions, potassium ions, calcium ions, and Xs each are independently selected from the group consisting of: 0, 1, 2, or 3 groups selected from the group consisting of: F, Cl, Br, I, -NH₂, -OH, -methoxyl, ethoxyl, propoxyl, isopropoxyl, -CN, methyl, ethyl, n-propyl, isopropyl, n-butyl, or isobutyl, or Xs each are independently a group obtained by substituting one or more groups selected from the group consisting of -O-, -CO-, and -NH₂ for any carbon atom on C₅-C₁₀ linear or branched alkyl.

14. The pharmaceutical composition according to any one of claims 9 to 13, **characterized in that** the compound of the Formula **I** is selected from the following compounds: 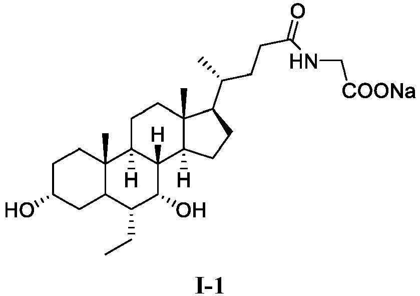 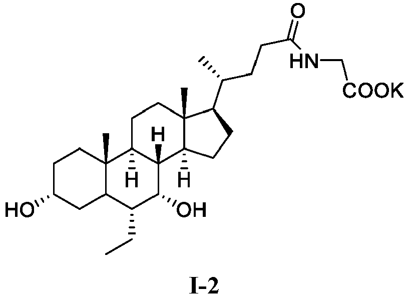 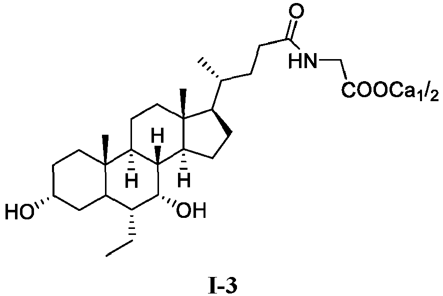 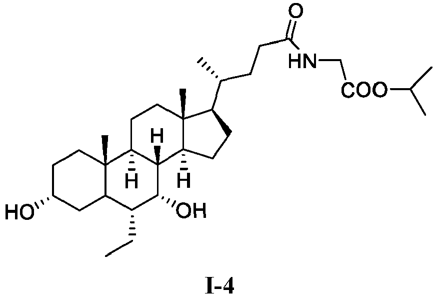 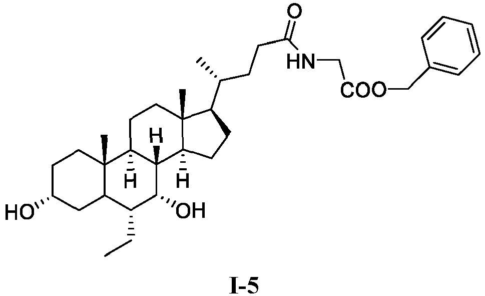 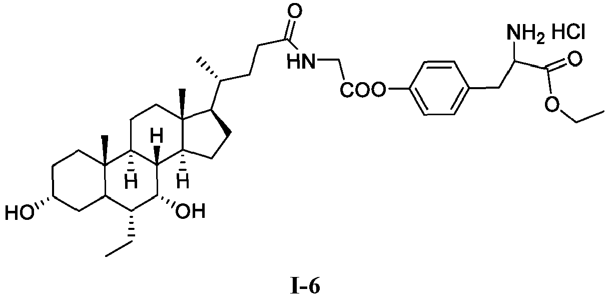 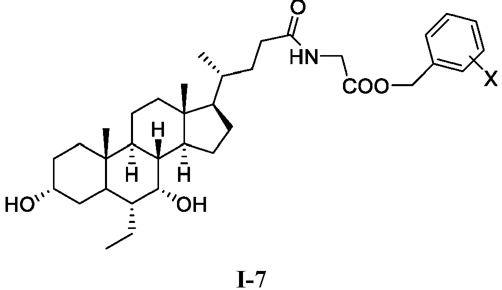
in Formula **I-7,** X is 1, 2, or 3 substituents, each independently selected from the group consisting of F, Cl, Br, I, -CN, -NH₂, -NO₂, or -OH, methyl, ethyl, propyl, isopropyl, methoxyl, ethoxyl, propoxyl, and isopropoxyl; and 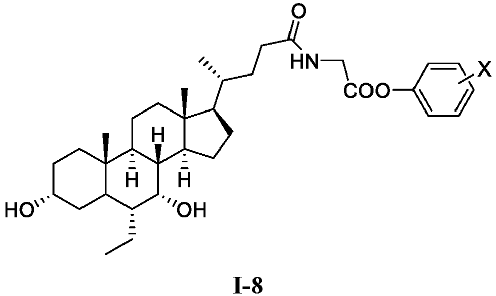
in Formula **I-8,** X is 1, 2, or 3 substituents, each independently selected from the group consisting of F, Cl, Br, I, -CN, -NH₂, -NO₂, or -OH, methyl, ethyl, propyl, isopropyl, methoxyl, ethoxyl, propoxyl, and isopropoxyl.

15. The pharmaceutical composition according to any one of claims 9 to 14, **characterized in that**: the pharmaceutically acceptable salt of the compound of Formula **I** includes a salt formed of the compound of Formula **I** and an acid; optionally, the acid includes an inorganic acid and an organic acid; optionally, the inorganic acid includes hydrochloric acid, hydrobromic acid, hydroiodic acid, sulfuric acid, nitric acid, phosphoric acid, and carbonic acid; and optionally, the organic acid includes formic acid, ascorbic acid, acetic acid, propionic acid, oxalic acid, malonic acid, succinic acid, fumaric acid, maleic acid, lactic acid, malic acid, citric acid, citric acid, tartaric acid, gluconic acid, hydrogen tartaric acid, glucuronic acid, carbonic acid, picric acid, methanesulfonic acid, ethanesulfonic acid, *p*-toluenesulfonic acid, benzoic acid, benzenesulfonic acid, *p*-bromobenzenesulfonic acid, glutamic acid, salicylic acid, and pamoic acid.

16. The pharmaceutical composition according to any one of claims 9 to 15, **characterized in that**: the dosage form of the pharmaceutical composition includes an oral preparation and an injection preparation;
optionally, the oral preparation includes a solid preparation and a liquid preparation;
optionally, the solid preparation includes tablets, powders, granules, and capsules; and
optionally, the liquid preparation includes water or oil suspensions, and syrups.

17. Use of the compound of Formula **I** or the pharmaceutically acceptable salt thereof according to any one of claims 1 to 8, or the pharmaceutical composition according to any one of claims 9 to 16 in the preparation of a medicament for treating nonalcoholic steatohepatitis.

18. Use of the compound of Formula **I** or the pharmaceutically acceptable salt thereof according to any one of claims 1 to 8, or the pharmaceutical composition according to any one of claims 9 to 16 in the preparation of a medicament for treating primary biliary cirrhosis.

19. Use of the compound of Formula **I** or the pharmaceutically acceptable salt thereof according to any one of claims 1 to 8, or the pharmaceutical composition according to any one of claims 9 to 16 in the preparation of a medicament for treating a biliation-associated disease;
optionally, the biliation-associated disease includes portal hypertension, bile acid diarrhea, alcoholic hepatitis, and primary sclerotic cholangitis.

20. A method for treating nonalcoholic steatohepatitis, **characterized in** administering, to a patient, a therapeutically effective amount of the compound of Formula **I** or the pharmaceutically acceptable salt thereof according to any one of claims 1 to 8, or the pharmaceutical composition according to any one of claims 9 to 16.

21. A method for treating primary biliary cirrhosis, **characterized in** administering, to a patient, a therapeutically effective amount of the compound of Formula **I** or the pharmaceutically acceptable salt thereof according to any one of claims 1 to 8, or the pharmaceutical composition according to any one of claims 9 to 16.

22. A method for treating a biliation-associated disease, **characterized in** administering, to a patient, a therapeutically effective amount of the compound of Formula **I** or the pharmaceutically acceptable salt thereof according to any one of claims 1 to 8, or the pharmaceutical composition according to any one of claims 9 to 16;
optionally, the biliation-associated disease includes portal hypertension, bile acid diarrhea, alcoholic hepatitis, and primary sclerotic cholangitis.
